# FASCICULE DE BREVET EUROPEEN

(11) **EP 1 440 075 B1**
(45) Date de publication et mention de la délivrance du brevet: **08.06.2005**
(21) Numéro de dépôt: 02785568.3
(22) Date de dépôt: 30.10.2002
(51) Int. Cl.: C07D 519/00, A61K 31/46, A61P 25/00, C07D 491/048, C07D 495/04

(54) **NOUVEAUX DERIVES AMIDES HETEROAROMATIQUES DE 3BETA-AMINO AZABICYCLOOCTANE, LEUR PROCEDE DE PREPARATION ET LEURS APPLICATIONS EN THERAPEUTIQUE**
NEUE HETEROAROMATISCHE AMIDDERIVATE VON 3BETA AMINOAZABICYCLOOCTANEN, VERFAHREN ZU IHRER HERSTELLUNG UND IHRE THERAPEUTISCHE VERWENDUNG
NOVEL 3BETA-AMINO AZABICYCLOOCTANE HETEROAROMATIC AMIDE DERIVATIVES, PREPARATION METHOD AND THERAPEUTIC USES THEREOF

(30) Priorité: 02.11.2001 FR 0114220
(43) Date de publication de la demande: 28.07.2004
(73) Titulaire: PIERRE FABRE MEDICAMENT, 92100 Boulogne-Billancourt (FR)
(72) Inventeur: IMBERT, Thierry, F-81290 Viviers-les-Montagnes (FR); MONSE, Barbara, 85358 Weichs (DE); KOEK, Wouter, San Antonio, TX 78232 (US)
(74) Mandataire: Callon de Lamarck, Jean-Robert
(86) Numéro de dépôt international: PCT/FR2002/003737
(87) Numéro de publication internationale: WO 2003/037904

(56) Documents cités:
- WO-A-97/10244
- ROMANELLI, M. N. ET AL: "Synthesis and biological activity of a series of aryl tropanyl esters and amides chemically related to 1H-indole-3-carboxylic acid endo 8-methyl-8-azabicyclo[3.2.1]oct-3-yl ester. Development of a 5-HT4 agonist endowed with potent antinociceptive activity" ARZNEIM.-FORSCH. (1993), 43(8), 913-18, XP002204702

## Description

La présente invention concerne les nouveaux dérivés de 3-hétéroaroyl amino-N-benzyl-8-aza-bicyclo(3.2.1)octane leur procédé de préparation et leur utilisation à titre de médicament.

Ces composés sont des agents antidopaminergiques, et utilisés ainsi en tant que médicaments antipsychotiques pour traiter la schizophrénie ou les troubles du système nerveux central sensible au traitement antidopaminergique, comme par exemple, les désordres obsessionnels compulsifs, l'anxiété, la dépression, l'autisme, les dyskinésies tardives, la toxicomanie et les troubles gastro-intestinaux.

La nécessité d'avoir une activité antidopaminergique, en particulier sur les récepteurs de la sous-classe D2, constitue une approche classique du traitement de la schizophrénie (Carlsson A., Am. J. Psychiatry, **135**, 164, 1978). Cependant, la plupart des composés ayant un tel mécanisme d'action ont l'inconvénient de présenter en clinique des effets secondaires indésirables, en particulier d'ordre extrapyramidaux (voir Acta Psychiatr. Scand. 1995, **91** (Suppl 388): 24-30) .

Le groupement 3β-amino tropane a été utilisé dans la série des benzamides pour fournir des composés doués d'activité antidopaminergique importante (EP 13138, US 4.536.580). Ces produits présentent à des degrés divers une activité antipsychotique, définie dans ces publications, mais cependant ne sont pas dénués d'effets secondaires cataleptigènes, et ne peuvent ainsi être utilisés en clinique. Des amides de l'amino-3β-nortropane ont été brevetés, par exemple WO 93/15052 décrit des composés antagonistes du canal calcique. Le brevet US 4,910,193 décrit des esters ou des amides pour le traitement des désordres gastrointestinaux induits par la sérotonine. Le brevet WO 01/14333 décrit des composés modulateurs du chimiotactisme eosinophilique. Aucun ne décrit l'utilisation de composés selon la formule générale pour le traitement de la schizophrénie. L'antériorité la plus proche est constituée par notre précédent brevet (FR 95/10655) décrivant des produits naphtamides de 3β-amino azabicyclo(3,2,1)octane. Ces produits sont des antagonistes puissants dopaminergiques et sérotoninergiques. Les produits de la présente invention se distinguent par le fait qu'ils sont beaucoup moins cataleptigènes encore, et présentent ainsi un meilleur profil d'activité.

L'article de Romanelli et al (Arzneim. - Forsch. (1993), 43(8), 913-18) décrit les esters aryle, propanyle et amide de l'acide 1H-indole-3-carboxylique endo de l'ester 8-methyl-8-azabicyclo[3.2.1]oct-3-yl comme ayant une activité analgésique. Toutefois, ce document ne décrit pas que de tels composés aient une activité anti-psychotique.

Il a été trouvé que les dérivés amides hétéroaromatiques fusionnés de 3-hétéroaroyl amino-N-benzyl-8-aza-bicyclo(3.2.1)octane, présentent à la fois une activité antipsychotique puissante, telle qu'elle peut être démontrée sur le test de l'inhibition des comportements induits par le méthylphénidate chez le rat, sans effets secondaires indésirables, et de plus sont dénués d'effets cataleptigènes (syndromes extrapyramidaux).

La présente invention a pour objet les nouveaux dérivés de 3-hétéroaroyl amino-N-benzyl-8-aza-bicyclo(3.2.1)octane, leur procédé de préparation, leur forme de sel pharmaceutiquement acceptable, les compositions pharmaceutiques les contenant et leur application en tant que médicament à usage de thérapeutique humaine, en particulier comme antipsychotiques.

Ces nouveaux composés répondent à la formule 1 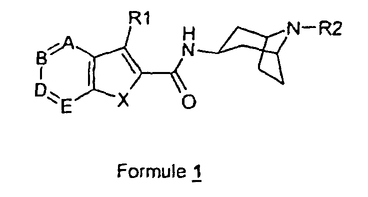 dans laquelle
A, B, D et E représentent un ou deux atomes d'azote , les autres étant des atomes de carbone.
X représente un S, ou un O, formant ainsi un système hétéroaromatique fusionné bicyclique tel que thièno-pyridine, thièno-pyrazine, furo-pyridine, furo-pyrazine.
R1 représente un groupe alcoxy en C1-C6, linéaire ou ramifié, un groupe alkyl thio en C1-C6, linéaire ou ramifié.
R2 représente un groupe alkyl linéaire, ramifié, cyclique en C2-C8, un groupe thiophèn-2 ou 3-ylméthyl, soit un groupe benzyl éventuellement substitué par un ou plusieurs substituants comme halogènes, F, Cl, Br, I, alkyl en C1-4, alcoxy en C1-C4, CF3, CN, NO2, OH.

De manière préférentielle le groupe hétéroaromatique constitue un squelette thièno[2,3-b]pyridine, le furo[2,3-b]pyridine, le thièno[3,2-b]pyridine, le furo[3,2-b]pyridine, le thièno[2,3-b]pyrazine, le furo[2,3-b]pyrazine, le groupe R1 est un méthoxy ou éthoxy, le groupe R2 est un cyclohexylméthyl, un thiophèn-2 ou 3-ylméthyl, ou un benzyl non substitué, ou substitué par un ou plusieurs F, Cl, OCH3, CN, et correspondent aux composés suivants:
3-Méthoxythièno[2,3-*b*]pyridine-2-carboxylic acid (8-benzyl-8-azabicyclo[3.2.1]oct-3β-yl)amide
3-Méthoxythièno[3,2-*b*]pyridine-2-carboxylic acid (8-benzyl-8-azabicyclo[3.2.1]oct-3β-yl)amide
3-Méthoxyfuro[3,2-*b*]pyridine-2-carboxylic acid (8-benzyl-8-azabicyclo[3.2.1]oct-3β-yl)amide
3-Méthoxythièno[3,2-*c*]pyridine-2-carboxylic acid (8-benzyl-8-azabicylo[3.2.1]oct-3β-yl)amide
3-Méthoxythièno[2,3-*b*]pyridine-2-carboxylic acid [8-(4-chlorobenzyl)-8-azabicyclo[3.2.1]oct-3β-yl] amide
3-Méthoxythièno[2,3-*b*]pyridine-2-carboxylic acid [8-(4-méthoxybenzyl)-8-azabicyclo[3.2.1]oct-3β-yl] amide
3-Méthoxythièno[2,3-*b*]pyridine-2-carboxylic acid [8-cyclohexylméthyl-8-azabicyclo[3.2.1]oct-3β-yl] amide
3-Méthoxythièno[2,3-*b*]pyridine-2-carboxylic acid [8-(4-fluorobenzyl)-8-azabicyclo[3.2.1]oct-3β-yl] amide
3-Méthoxythièno[2,3-*b*]pyridine-2-carboxylic acid [8-thiophèn-3-ylméthyl-8-azabicyclo[3.2.1]oct-3β-yl] amide
3-Méthoxythièno[2,3-c]pyridine-2-carboxylic acid (8-benzyl-8-azabicyclo[3.2.1]oct-3β-yl]amide
3-Isopropoxythièno[2,3-*b*]pyridine-2-carboxylic acid (8-benzyl-8-azabicyclo[3.2.1]oct-3β-yl]amide
3-Éthoxythièno[2,3-*b*]pyridine-2-carboxylic acid (8-benzyl-8-azabicyclo[3.2.1]oct-3β-yl)amide
3-Méthoxyfuro[3,2-*b*]pyridine-2-carboxylic acid [8-(4-fluorobenzyl)-8-azabicyclo[3.2.1]oct-3β-yl]amide
3-Méthoxyfuro[3,2-*b*]pyridine-2-carboxylic acid [8-(4-chlorobenzyl)-8-azabicyclo[3.2.1]oct-3β-yl]amide
3-Méthoxyfuro[3,2-*b*]pyridine-2-carboxylic acid (8-cyclohexylméthyl-8-azabicyclo[3.2.1]oct-3β-yl)amide
3-Méthylsulfanylthièno[2,3-*b*]pyridine-2-carboxylic acid (8-benzyl-8-azabicyclo[3.2.1]oct-3β-yl)amide
3-Méthoxythièno[2,3-*b*]pyrazine-2-carboxylic acid (8-benzyl-8-azabicyclo[3.2.1]oct-3β-yl)amide
3-Méthoxythièno[2,3-*b*]pyrazine-2-carboxylic acid [8-(4-chlorobenzyl)-8-azabicyclo[3.2.1]oct-3β-yl]amide
3-Méthylsulfanylthièno[2,3-*b*]pyrazine-2-carboxylic acid (8-benzyl-8-azabicyclo[3.2.1]oct-3β-yl)amide
3-Méthylsulfanylthièno[2,3-*b*]pyrazine-2-carboxylic acid [8-(4-chlorobenzyl)-8-azabicyclo[3.2.1]oct-3β-yl]amide
3-Méthoxythièno[2,3-*b*]pyrazine-2-carboxylic acid [8-(4-fluorobenzyl)-8-azabicyclo[3.2.1]oct-3β-yl]amide
3-Méthylsulfanylthièno[2,3-*b*]pyrazine-2-carboxylic acid [8-(4-fluorobenzyl)-8-azabicyclo[3.2.1]oct-3β-yl]amide
3-Méthoxythièno[2,3-*b*]pyrazine-2-carboxylic acid (8-thiophèn-2-ylméthyl-8-azabicyclo[3.2.1]oct-3β-yl)amide
3-Méthoxyfuro[2,3-*b*]pyrazine-2-carboxylic acid (8-benzyl-8-azabicyclo[3.2.1]oct-3β-yl)amide
3-Méthoxyfuro[2,3-*b*]pyrazine-2-carboxylic acid [8-(4-chlorobenzyl)-8-azabicyclo[3.2.1]oct-3β-yl]amide
3-Méthoxyfuro[2,3-*b*]pyrazine-2-carboxylic acid [8-(4-fluorobenzyl)-8-azabicyclo[3.2.1]oct-3β-yl]amide.

Les produits de la présente invention sont des ligands des récepteurs dopaminergiques centraux de type D2. La mise en évidence de leurs propriétés est faite sur la base de leur affinité sur les récepteurs dopaminergiques, par déplacement du ligand radioactif (³H-YM-09151-2) qui marque spécifiquement ces récepteurs. Ce procédé d'étude de liaisons spécifiques est décrit dans Naunyn-Schmiedeberg's Arch.Pharmacol. **21**, 301-314, 1982.

A titre d'exemple, les valeurs sont indiquées dans le Tableau I suivant, en comparaison des substances de référence.

**Tableau I**

| Composés: | Exemples N° | Affinité D2 (pKi) |
|---|---|---|
| Halopéridol | | 9.01 |
| Sulpiride | | 8.39 |
| Risperidone | | 8.7 |
| Spiperone | | 10 |
| Tropapride | | 10.02 |
| | 32 | 9.17 |
| | 29 | 9.18 |
| | 31 | 8.69 |
| | 27 | 9.36 |
| | 2 | 9.35 |
| | 11 | 9.11 |
| | 10 | 8.59 |
| | 12 | 8.59 |
| | 8 | 8.76 |
| | 4 | 8.69 |

Les valeurs de l'affinité sur les récepteurs D2 sont comparables à celles des produits de référence. La puissance de cette affinité est nécessaire pour obtenir l'activité *in vivo* recherchée, mesurée par la normalisation des comportements induits par le méthylphénidate, comme il est indiqué plus loin.

Les composés ont été testés in vivo (Tableau II) pour mesurer leur capacité à antagoniser les comportements induits par le méthylphénidate chez le rat. Ce test très discriminant permet de sélectionner des composés qui se révèlent actifs chez l'homme comme antipsychotiques comme cela a été montré dans: W. Koek, F. C. Colpaert J. Pharmacol. Exp. Ther. **267**, 181 (1993). Ce test caractérise l'activité antipsychotique de manière plus approfondie que l'antagonisme des effets de l'apomorphine, test classiquement utilisé.

**Tableau II**

| N° de l'exemple | Inhibition du mâchonnement ED50 (mg/kg) | Normalisation ED50 (mg/kg) | Catalepsie ED50 (mg/kg)* | Ratio: Catalepsie/Normalisation |
|---|---|---|---|---|
| 2 | 0.08^{a} | 0.56 ^{a} | 1.8 ^{a} | 3.2 |
| 8 | 0.10^{a} | 1.3 ^{a} | >40 ^{a} | >30 |
| 10 | 1.25^{a} | 5 ^{a} | >40 ^{a} | >8 |
| 11 | 0.08 ^{a} | 0.31 ^{a} | >40 ^{a} | > 129 |
| 12 | 1.25 ^{a} | 1.25 ^{a} | >40 ^{a} | >32 |
| 4 | 0.12 ^{a} | 0.98 ^{a} | >40 ^{a} | >40 |
| 32 | 0.08 ^{b} | 0.31 ^{b} | 1.25 ^{b} | 4 |
| 29 | 0.08 ^{b} | 0.32 ^{b} | 5 ^{b} | 15.6 |
| 31 | 0.31 ^{b} | 0.31 ^{b} | 1.25 ^{b} | 4 |
| 27 | 0.27 ^{b} | 0.67 ^{a} | 9.5 ^{b} | 14 |
| Risperidone | 0.25 ^{a} | 2.8 ^{a} | 1.2 ^{a} | 0.4 |
| Tropapride | 0.0059 ^{a} | 0.017 ^{a} | 0.015 ^{a} | 0.9 |
| Haloperidol | 0.08 ^{a} | 0.32 ^{a} | 0.32 ^{a} | 1 |
| Sulpiride | >40 ^{a} | >40 ^{a} | > 160 ^{a} | |

| | | | | |
|---|---|---|---|---|
| Résultats sur une expérience de 3 ou 5 animaux. ^{a} i.p. | | | | |
| ^{b} p.o. | | | | |

Le tropapride est pris en comparaison comme produit de référence à cause de sa structure chimique tropane (Drugs of the Future vol 9, n°9, 673, 1984)

Les résultats obtenus montrent que les composés de l'invention sont capables non seulement d'inhiber le mâchonnement stéréotypé, mais aussi de normaliser tous les comportements induits par le méthylphénidate, et ceci en l'absence d'effets secondaires indésirables.

De plus, les composés de la présente invention sont évalués pour leur activité cataleptigène selon le protocole décrit dans : Eur. J. Pharmacol. **356**, 189 (1998). Le Tableau II montre que les produits de référence manifestent une activité cataleptigène aux doses où ils normalisent les comportements induits par le méthylphénidate.

Les composés de la présente invention, au contraire, ne montrent que peu ou pas de catalepsie quand ils sont évalués seuls chez le rat en l'absence de méthylphénidate. Certains composés, contrairement aux produits de référence, montrent un écart entre la dose cataleptigène et la dose induisant la normalisation, et bien plus, d'autres ne montrent aucune catalepsie aux doses étudiées, comme il est indiqué par le ratio : catalepsie/normalisation (Tableau II). Ainsi les produits de la présente invention se positionnent de façon extrêmement favorable par rapport aux composés de référence.

Il ressort de notre étude, et c'est l'objet de l'invention, que les produits ici décrits sont doués d'une activité antipsychotique importante, sans provoquer d'effets secondaires indésirables, ni de catalepsie aux doses utilisées. Ceci laisse présager une faible propension de ces produits à induire chez l'homme les effets extrapyramidaux néfastes que l'on observe avec de nombreux produits classiquement utilisés. Ces paramètres biologiques *in vivo* constituent les caractéristiques des produits de l'invention qui se distinguent de ceux classiquement utilisés, et ainsi permettent de résoudre le problème médical du traitement de la schizophrénie. Le confort du patient est ainsi accru au cours du traitement, et la manipulation de ces produits pour le traitement des troubles psychotiques par le clinicien s'en trouve ainsi bien amélioré par rapport aux produits usuels, la marge thérapeutique en est également améliorée.

La présente invention concerne donc les composés de formule générale **1** comme médicament utiles en particulier dans le traitement de la schizophrénie.

Les composés de la présente invention peuvent former des sels par addition d'acide minéral ou organique pharmaceutiquement acceptable et entrer dans des compositions pharmaceutiques pour pouvoir être administrées par les différentes voies usuelles, formes orales, injectables, parentérales.

La présente invention concerne également l'utilisation de ces composés de formule **1** entrant dans une composition pharmaceutique de formulation correspondante a son mode d'administration, comprimés, capsules, gélules, adaptées à la clinique humaine et à des doses quotidiennes comprises entre 0.1 et 500 mg ou plus spécifiquement 0.1 à 100 mg de principe actif.

Les produits de la présente invention sont obtenus par analogie avec des procédés connus dont l'étape clé est la formation de la fonction amide entre l'acide hétéroaromatique de formule **2** et l'amine de formule **3****,** selon le Schéma I:

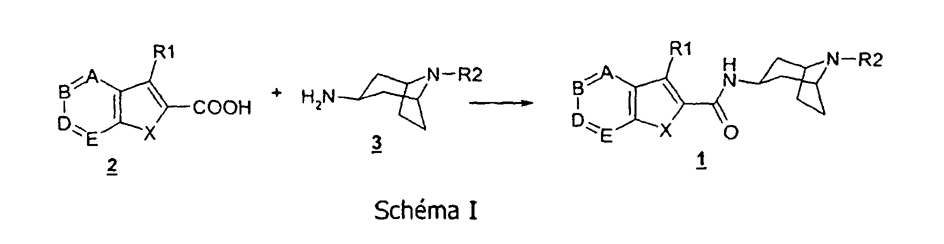

La liaison amide se forme par activation de l'acide **2** (c.à.d. anhydride mixte, chlorure d'acide) avec une amine **3** dans un solvant inerte (dichlorométhane, THF) en présence d'une base (c.à.d. une amine comme la pyridine ou la triéthylamine) de 0°C à 100°C de préférence à 0°C.

Les conditions de couplage impliquent l'action du chloroformiate d'alkyle en présence de triéthylamine, dans le chlorure de méthylène, à basse température ou bien l'action du chlorure d'acide de l'acide hétérocyclique correspondant.

Les accès aux systèmes hétérocycliques acides **2** sont décrits dans la littérature chimique.

Le 3-hydroxy-2-méthoxycarbonylthièno[3,2-b]pyridine, le 3-hydroxy-2-méthoxycarbonylthièno[2,3-b]pyridine, le 3-hydroxy-2-méthoxycarbonylthièno[3,2-c]pyridine, le 3-hydroxy-2-méthoxycarbonylthièno[2,3-c]pyridine sont décrits dans Journal of Hétérocyclic Chemistry 1987, **24**, 85. Le 3-hydroxy-2-éthoxycarbonylfuro[2,3-c]pyridine est décrit dans Journal of Hétérocyclic Chemistry, 1986, **23**, 549. Le 3-hydroxy-2-éthoxycarbonylfuro[3,2-b]pyridine est décrit dans Journal of Hétérocyclic Chemistry, 1986, **23**, 665. Le 3-hydroxy-2-éthoxycarbonylfuro[2,3-b]pyridine est décrit dans Journal of Hétérocyclic Chemistry, 1986, **23**, 1465. Le 3-hydroxy-2-éthoxycarbonylfuro[3,2-c]pyridine est décrit dans Journal of Hétérocyclic Chemistry, 1988, **25,** 1205.

Les produits hétérocycliques précédents possédant une fonction 3-hydroxy peuvent être alkylés dans des conditions classiques, comme le diméthylsulfate ou un iodure d'alkyle , dans l'acétonitrile ou l'acétone en présence de carbonate de potassium. La saponification de la fonction ester en position 2 par KOH ou NaOH dans un alcool livre les acides du type **2**.

Les composés possédant un groupement méthylthio en position 3 sont obtenus par substitution d'un groupe partant, comme un phosphate en position 3 du système hétérocyclique, par un thiol comme un alkyl mercaptan, dans un solvant inerte. Cette méthode est décrite dans le brevet WO 9313664.

Le 3-hydroxy-2-méthoxycarbonylthièno[2,3-b]pyrazine est obtenue à partir du 2-chloro-3-méthoxycarbonylpyrazine, lui même décrit dans Journal of the Chemical Society 1996, 247-254. Le chlore en position 2 de cette pyrazine est substitué par le thioglycolate de méthyle en milieu basique, et l'adduit obtenu est cyclisé dans la même réaction en 3-hydroxy-2-méthoxycarbonylthièno[2,3-b]pyrazine. Le 3-hydroxy-2-méthoxycarbonylfuro[2,3-b]pyrazine est obtenu par la même transformation en utilisant alors le glycolate de méthyle.

La diamine de formule **3** (Schéma I) est décrite dans Eur. J. Med. Chem. 1984, **19**, 105. Elle possède un substituant R2, qui peut être un groupe benzyl substitué. La débenzylation par hydrogénolyse sur charbon palladié impose de protéger auparavant l'amine primaire par un groupement protecteur de type carbamate comme le terbutoxycarbonyl, clivable ultérieurement en milieu acide. L'alkylation de l'amine secondaire issue de l'hydrogénolyse, l'amine primaire étant protégée, se fait de façon classique et univoque avec les différents halogénures de benzyle substitués. De meilleurs rendements sont obtenus par alkylation dans la méthyléthyl cétone au reflux, en présence de carbonate de césium, et adjonction de traces d'iodure de potasium. Il est également possible de débenzyler le composé de la formule générale **1** quand R2 est un groupe benzyl, par le formiate d'ammonium en présence d'hydroxyde de palladium sur charbon, en milieu hydroalcoolique. Les amines **3** peuvent être obtenues également par amination réductrice d'aldéhydes aromatiques telles que le 2-thiophèncarboxaldéhyde, avec le *ter*-butyl-N-(8-azabicyclo[3.2.1]oct-3β-yl)carbamate, avec le triacétoxyborohydrure de sodium dans l'acide acétique.

Font également partie de la présente invention les composés de formule **4**, comme intermédiaires de synthèse, dans laquelle X est un O ou S, R1 un OH, un alcoxy en C1-6, ou un thioalcoxy en C1-6, et R représente un hydrogène, sauf dans le cas où R1 est OH, un méthyl ou un éthyl.

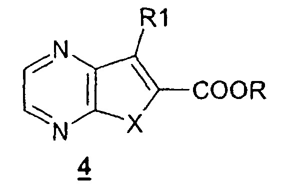

Les composés de formule 4 préférés sont choisis parmi :
3-Hydroxy-2méthoxycarbonylfuro[2,3-b]pyrazine
3-méthylsulfanyl-2méthoxycarbonylfuro[2,3-b]pyrazine
acide 3-méthoxy-furo[2,3-b]pyrazine-2-carboxylique
acide 3-éthoxy-furo[2,3-b]pyrazine-2-carboxylique
acide 3-méthylsulfanylfuro[2,3-b]pyrazine-2-carboxylique
3-Hydroxy-2méthoxycarbonylthièno[2,3-b]pyrazine
3-méthylsulfanyl-2méthoxycarbonylthièno[2,3-b]pyrazine
acide 3-méthoxythièno[2,3-b]pyrazine-2-carboxylique
acide 3-éthoxythièno[2,3-b]pyrazine-2-carboxylique
acide 3-méthylsulfanylthièno[2,3-b]pyrazine-2-carboxylique

Ces produits se préparent par action d'esters de l'acide glycolique sur la 3-chloro-2-pyrazine carboxylate de méthyle, en milieu basique.

Les exemples suivants illustrent l'invention:

### Exemple 1:

3-Méthoxythièno[2,3-*b*]pyridine-2-carboxylic acid (8-benzyl-8-azabicyclo[3.2.1]oct-3β-yl)amide chlorhydrate

### a) 3-Méthoxy-2-méthoxycarbonylthièno[2,3-b]pyridine

On chauffe à reflux 995 mg du 3-hydroxy-2-méthoxycarbonylthièno[2,3-*b*]pyridine (4.76 mmol, 1éq) pendant 4h avec 0.54 ml de diméthylsulfate (5.71mmol, 1.2éq) dans 30ml d'acétone en présence de 987 mg de carbonate de potassium (7.14 mmol, 1.5éq). On filtre l'insoluble, on lave avec de l'acétone et on évapore sous vide.
On obtient 600 mg du produit (56%) après filtration rapide sur silice.
RMN (¹H, CDCl₃) : 3.90 (s; 3H, OCH₃), 4.15 (s; 3H, OCH₃), 7.31 (dd, J = 8.3, 4.7Hz; 1H), 8.12 (dd, J = 8.3, 1.5Hz; 1H), 8.67 (dd, J = 4.7, 1.5Hz; 1H).

### b) Acide 3-méthoxythièno[2,3-b]pyridine-2-carboxylique

On chauffe à reflux 590 mg de 3-méthoxy-2-méthoxycarbonylthièno[2,3-*b*]pyridine (2.64mmole, 1éq) obtenu précédemment au stade a), pendant 1h avec 4.0 ml de NaOH (4.0mmol, 1.5éq) dans 10 ml d'éthanol. On laisse revenir à température ambiante, on dilue par l'eau et on neutralise par 4.5 ml d'HCl 1N. On filtre le précipité formé, on lave par l'eau et on sèche.
On obtient 500 mg du produit (91%).
RMN (¹H, DMSO-d₆) : 4.10 (s; 3H, OCH₃), 7.53 (dd, J = 8.1, 4.5Hz; 1H), 8.30 (dd, J = 8.1, 1.4Hz; 1H), 8.74 (dd, J = 8.1, 1.4Hz; 1H), 13.55 (large; 1H, COOH)

### c) 3-Méthoxythièno[2,3-b]pyridine-2-carboxylic acid (8-benzyl-8-azabicyclo[3.2.1]oct-3β-yl)amide chlorhydrate

On additionne 0.25 ml de chloroformiate d'éthyle (2.6 mmol, 1.1éq) dans 50ml de dichlorométhane à une solution de 496 mg d'acide 3-méthoxythièno[2,3-*b*]pyridine-2-carboxylique (2.4mmole, 1éq) obtenu au stade précédent au stade b), dans 100 ml de dichlorométhane à 0°C en présence de 0.4 ml de triéthylamine (2.8mmol, 1.2éq). Après 15min, on introduit goutte à goutte une solution de 487 mg de 8-(phénylméthyl)-8-azabicyclo[3.2.1]octane-3β-amine (2.25 mmol, 0.95éq) dans 50 ml de dichlorométhane. Ensuite, on laisse agiter à température ambiante jusqu'à consommation totale des produits de départ. On lave trois fois par l'eau, on sèche et on évapore le solvant.

On obtient 455 mg du produit (47%) après chromatographie flash sur silice et chromatographie sur alumine.

### Préparation du sel:

On dissout 455 mg de la base (1.1 mmol, 1éq) obtenue dans 5 ml d'éthanol. On y introduit 0.6 ml d'une solution d'isopropanol - HCl 3.6N (2.2 mmol, 2éq). On évapore sous vide, on reprend dans d'acétate d'éthyle, on filtre le sel formé et on sèche.
Rdt.: 368 mg (85%).
PF = 228°C
IR : 3355, 1645, 1540
RMN (base ; ¹H, CDCl₃) : 1.65 (m ; 2H), 1.78 (m ; 2H), 1.95 (m ; 2H), 2.09 (m ; 2H), 3.29 (m ; 2H, H1 et H5) 3.55 (s ; 2H, N- CH₂-Ph), 4.27 (s ; 3H, OCH₃), 4.40 (m ; 1H, H3), 7.26 (m ; 2H), 7.35 (m ; 3H), 7.39 (m ; 2H), 8.08 (dd, J = 8.1, 1.3Hz ; 1H), 8.65 (dd, J = 4.5, 1.3Hz ; 1H)

| | | |
|---|---|---|
| Analyse | C₂₃ H₂₅ N₃ O₂ S | Masse = 407.54 |
| | C₂₃ H₂₅ N₃ O₂ S, HCl | Masse = 444.00 |
| | C₂₃ H₂₅ N₃ O₂ S, HCl , 0.1 H₂O | Masse = 445.80 |

| | %C | %H | %N | %H₂O |
|---|---|---|---|---|
| calc. | 61.97 | 5.92 | 9.43 | 0.4 |
| trouv. | 61.98 | 5.95 | 9.47 | 0.3 |

### Exemple 2:

3-Méthylsulfanylthièno[2,3-*b*]pyridine-2-carboxylic acid (8-benzyl-8-azabicyclo[3.2.1]oct-3β-yl)amide dichlorhydrate

### a) 3-(Diéthoxyphosphoryloxy)thièno[2,3-b]pyridine-2-carboxylic acid méthyl ester

On additionne 261mg de NaH (60% dans l'huile minérale ; 6.54 mmol, 1.2éq) à une suspension de 1.14g de 3-hydroxy-2-méthoxycarbonylthièno[2,3-*b*]pyridine (5.45mmole, 1éq) (obtenu selon J.Hétéroc.Chem. 1987, **24**, 85) dans 20ml de tétrahydrofuranne à 0°C sous azote. Après 15 min, on y introduit une solution de 0.78 ml de diéthylphosphorochlorhydate (5.45 mmol, 1éq) dans 10 ml de tétrahydrofuranne. Ensuite, on laisse le mélange réactionnel agiter à température ambiante pendant 18h. On verse le milieu réactionnel sur 100 ml d'eau glacée, on acidifie par HCl 1N et on extrait 3 fois par l'acétate d'éthyle. On sèche la phase organique sur Na₂SO₄ et on évapore sous vide. On obtient 750 mg (40 %) de produit purifié par chromatographie flash sur silice (éluant: acétate d'éthyle / éther de pétrole = 2 /3).
RMN (¹H; CDCl₃): 1.37 (t, J = 7.2Hz ; 6H), 3.95 (s ; 3H), 4.32 (q, J = 7.2Hz ; 4H), 7.39 (dd, J = 8.2, 4.6Hz ; 1H), 8.42 (dd, J = 8.2, 1.6Hz ; 1H), 8.71 (dd, J = 4.6, 1.6Hz ; 1H)

### b) 3-Méthylsulfanylthièno[2,3-b]pyridine-2-carboxylic acid méthyl ester

On additionne une solution de 736 mg du produit du stade a) précédent (2.13 mmol, 1éq) dans 15 ml de tétrahydrofuranne à une suspension de 300 mg de sodium thiométhoxide (4.26 mmol, 2éq) dans 5 ml de tétrahydrofuranne à 0°C sous azote. Ensuite, on laisse le milieu réactionnel agiter à température ambiante pendant 5 jours. On additionne 10 ml d'eau, on acidifie par l'HCl 1N et on extrait 3 fois par l'acétate d'éthyle. On sèche la phase organique sur Na₂SO₄ et on évapore sous vide.

On obtient 270 mg (55%) de produit après chromatographie flash sur silice (éluant: acétate d'éthyle / éther de pétrole = 1 / 9).
RMN (¹H, CDCl₃) : 2.50. (s ; 3H), 3.86 (s ; 3H), 7.52 (dd, J = 8.1, 4.6Hz ; 1H), 8.36 (dd, J = 8.1, 1.6Hz ; 1H), 8.74 (dd, J = 4.6, 1.7Hz ; 1H)

### c) 3-Méthylsulfanylthièno[2,3-b]pyridine-2-carboxylic acid

On chauffe à reflux 270 mg de produit du stade b) précédent (1.1mmole, 1éq) pendant 18h dans 20 ml d'un mélange 50/50 H₂O/méthanol en présence de 1.7 ml de NaOH 1N (1.7 mmol, 1.5éq). On laisse revenir à température ambiante, on ajoute de l'eau et on additionne 2 ml d'HCl 1N. On filtre le précipité formé, on lave par l'eau et on sèche.

On obtient 242 mg de l'acide (95%).
PF = 214°C
RMN (¹H, DMSO-d₆) : 2.51 (s ; 3H), 7.60 (dd, J = 8.1, 4.5Hz ; 1H), 8.46 (dd, J = 8.1, 1.5Hz ; 1H), 8.77 (dd, J = 4.5, 1.5Hz ; 1H), 13.92 (s ; large, 1H)

### d) 3-Méthylsulfanylthièno[2,3-b]pyridine-2-carboxylic acid (8-benzyl-8-azabicyclo[3.2.1]oct-3β-yl)amide dichlorhydrate

Ce composé est obtenu selon la même méthode que pour l'exemple 1 c).
PF = 236°C
IR : 1637, 1534
RMN (base; ¹H, CDCl₃) : 1.72 (m ; 2H), 1.78 (m ; 2H), 1.98 (m ; 2H), 2.10 (m ; 2H), 2.41 (s ; 3H, SCH₃), 3.31 (m ; 2H, H1 et H5), 3.57 (s ; 2H, N-CH₂-Ph), 4.43 (m ; 1H, H3), 7.26 (m ; 2H), 7.34 (t, J = 7.2Hz ; 2H), 7.41 (m ; 3H), 8.26 (dd, J = 8.2, 1.6Hz ; 1H), 8.58 (d, J = 8.0Hz ; 1H, NH), 8.68 (dd, J = 4.6, 1.6Hz ; 1H)

| | | |
|---|---|---|
| Analyse | C₂₃ H₂₅ N₃ O S₂ | Masse = 423.60 |
| | C₂₃ H₂₅ N₃ O S₂, 2 HCl | Masse = 496.52 |
| | C₂₃ H₂₅ N₃ O S₂, 2 HCl, 0.5 H₂O | Masse = 505.53 |

| | %C | %H | %N | %H₂O |
|---|---|---|---|---|
| calc. | 54.65 | 5.58 | 8.31 | 1.8 |
| trouv. | 54.67 | 5.55 | 8.22 | 2.2 |

SM : M⁺ = 423.9 (100%)

### Exemple 3:

3-Méthoxythièno[3,2-*b*]pyridine-2-carboxylic acid (8-benzyl-8-azabicyclo[3.2.1]oct-3β-yl)amide dichlorhydrate

### a) 3-Bromo-2-picoline

Ce composé est obtenu selon la méthode décrite dans J. Med. Chem. 1987, **30**, 877.
RMN (¹H, CDCl₃) : 2.67 (s ; 3H), 7.01 (dd, J = 7.9, 4.7Hz ; 1H), 7.80 (dd, J = 7.9, 1.0Hz ; 1H), 8.43 (dd, J = 4.7, 1.0Hz ; 1H)

### b) 3-Bromo-2-pyridinecarboxylic acid.

Ce composé est obtenu selon la méthode décrite dans J. Med. Chem. 1974, **17**, 1065.
RMN (¹H, DMSO-d₆) : 5.75 (large), 7.48 (dd, J = 8.1, 4.6Hz ; 1H), 8.20 (dd, J = 8.1, 1.0Hz ; 1H), 8.59 (dd, J = 4.6, 1.0Hz ; 1H)

### c) 3-Bromo-2-pyridinecarboxylic acid méthyl ester

On chauffe à reflux un mélange de 5.45 g du 3-bromo-2-pyridinecarboxylic acid (27.0mmole, 1éq), obtenu à l'exemple 3 b), dans 100 ml du méthanol en présence de 8 ml d'acide sulfurique. On laisse revenir à température ambiante et on verse le mélange réactionnel dans l'eau. On extrait 3 fois par l'acétate d'éthyle, on sèche la phase organique sur Na₂SO₄ et on évapore sous vide. On obtient 2.48g (42%) du produit estérifié.
RMN (¹H, CDCl₃) : 4.02 (s ; 3H), 7.34 (dd, J = 8.2, 2.8Hz ; 1H), 8.04 (d, J = 8.2Hz ; 1H), 8.64 (d, J = 2.8Hz ; 1H)

### d) 3-Hydroxythièno[3,2-b]pyridine-2-carboxylic acid méthyl ester

Ce composé est décrit dans J. Hétérocycl.Chem. 1987, **24**, 85.

On chauffe à reflux un mélange de 2.48 g du produit obtenu au stade précédent exemple 3 c) (11.0 mmol, 1éq) avec 1.03 ml du méthyl thioglycolate (11.0 mmol, 1éq) dans 100 ml de l'acétonitrile en présence de 2.4 g du carbonate de potassium (17.0 mmol, 1.5éq). Ensuite, on laisse revenir à température ambiante, on évapore le solvant sous vide et on reprend le résidu dans l'eau. On additionne l'acide acétique (pH 4) et on filtre le précipité formé.

On obtient 852 mg du produit (35%).
PF = 184°C
RMN (¹H, CDCl₃) : 4.00 (s ; 3H), 7.42 (dd, J = 8.2, 4.4Hz ; 1H), 8.11 (dd, J = 8.2, 1.3Hz ; 1H), 8.79 (dd, J = 4.4, 1.3Hz ; 1H), 9.96 (large ; 1H)

### e) 3-Méthoxythièno[3,2-b]pyridine-2-carboxylic acid méthyl ester

La méthylation de l'hydroxy se fait selon le mode opératoire de l'exemple 1 a).
RMN (¹H, CDCl3) : 3.96 (s ; 3H), 4.42 (s ; 3H), 8.37 (dd, J = 8.3, 4.4Hz ; 1H), 8.10 (dd, J = 8.3, 1.3Hz ; 1H), 8.75 (dd, J = 4.5, 1.3Hz ; 1H), 13.56 (large ; 1H)

### f) 3-Méthoxythièno[3,2-b]pyridine-2-carboxylic acid (8-benzyl-8-azabicyclo[3.2.1]oct-3β-yl)amide dichlorhydrate

Ce composé est obtenu selon la même méthode que pour l'exemple 1 b) et 1 c).
PF= 239°C
IR : 3399, 1659, 1535
RMN (base ; ¹H, CDCl₃) : 1.68 (m ; 2H), 1.78 (m ; 2H), 1.96 (m ; 2H), 2.09 (m ; 2H), 3.29 (m ; 2H, H1 et H5), 3.56 (s ; 2H, N-CH₂-Ph), 4.38 (m ; 1H, H3), 4.47 (s ; 3H, OCH₃), 7.26-7.47 (m ; 6H), 8.10 (d, J = 7.2Hz ; 1H), 8.68 (d, J = 3.0Hz ; 1H)

| | | |
|---|---|---|
| Analyse | C₂₃ H₂₅ N₃ O₂ S | Masse = 407.56 |
| | C₂₃ H₂₅ N₃ O₂ S, 2 HCl | Masse = 480.46 |
| | C₂₃ H₂₅ N₃ O₂ S, 2 HCl, 0.5 H₂O | Masse = 489.47 |

| | % C | %H | %N | % H₂O |
|---|---|---|---|---|
| calc. | 56.44 | 5.77 | 8.58 | 1.8 |
| trouv. | 54.71 | 5.47 | 8.28 | 1.5 |

SM (DCI, NH₃) : 408 (M⁺, 100%)

### Exemple 4:

3-Méthoxyfuro[3,2-*b*]pyridine-2-carboxylic acid (8-benzyl-8-azabicyclo[3.2.1]oct-3β-yl)amide dichlorhydrate

### a) 3-Hydroxypyridine-2-carboxylic acid éthyl ester

Ce composé est préparé selon Tet. Lett. 1996, **27**, 459.
RMN (¹H, CDCl₃) : 1.49 (t, J = 7.2Hz ; 3H), 4.54 (q, J = 7.2Hz ; 2H), 7.37 (dd, J = 8.5, 1.4Hz ; 1H), 7.42 (dd, J = 8.5, 5.4Hz ; 1H), 8.30 (dd, J = 4.1, 1.4Hz ; 1H), 10.78 (s ; 1H)

### b) 3-Éthoxycarbonylméthoxypyridine-2-carboxylic acid éthyl ester

Ce composé est préparé selon J.Hétérocycl.Chem. 1986, **23**, 665. RMN (¹H, CDCl₃) : 1.29 (t, J = 7.0Hz ; 3H), 1.44 (t, J = 7.0Hz ; 3H), 4.26 (q, J = 7.0Hz ; 2H), 4.47 (q, J = 7.0Hz ; 2H), 4.74 (s ; 2H), 7.29 (d, J = 8.4Hz ; 1H), 7.39 (dd, J = 8.4, 3.6Hz ; 1H), 8.35 (d, J = 3.6Hz ; 1H)

### c) 3-Hydroxyfuro[3,2-b]pyridine-2-carboxylic acid éthyl ester

Ce composé est préparé selon J.Hétérocycl.Chem. 1986, **23**, 665. PF= 190°C
RMN (¹H, CDCl₃) : 1.47 (t, J = 7.2Hz ; 3H), 4.50 (q, J = 7.2Hz ; 2H), 7.43 (dd, J = 8.6, 4.6Hz ; 1H), 7.81 (d, J = 8.6Hz ; 1H), 8.68 (d, J = 8.6Hz ; 1H)

### d) 3-Méthoxyfuro[3,2-b]pyridine-2-carboxylic acid éthyl ester

La méthylation de l'hydroxy se fait selon le mode opératoire de l'exemple 1 a).
RMN (¹H, CDCl₃) : 1.44 (t, J = 7.2Hz ; 3H), 1.47 (q, J = 7.2Hz ; 1H), 4.57 (s ; 3H), 7.38 (dd, J = 8.5, 4.6Hz ; 1H), 7.80 (dd, J = 8.5, 1.0Hz ; 1H), 8.61 (dd, J = 4.6, 1.0Hz 1H)

### e) 3-Méthoxyfuro[3,2-b]pyridine-2-carboxylic acid

Ce composé est obtenu par le même mode opératoire que pour l'exemple 1 b).
PF = 207°C
RMN (¹H, DMSO-d₆) : 4.42 (s ; 3H), 7.56 (dd, J = 8.6, 4.6Hz ; 1H), 8.12 (dd, J = 8.6, 1.0Hz ; 1H), 8.65 (dd, J = 4.6, 1.0Hz ; 1H), 13.39 (large ; 1H)

### f) 3-Méthoxyfuro[3,2-b]pyridine-2-carboxylic acid (8-benzyl-8-azabicyclo[3.2.1]oct-3β-yl)amide dichlorhydrate

Ce composé est obtenu selon la même méthode que pour l'exemple et 1 c).
PF = 172°C
IR : 3420, 3048, 1732, 1665
RMN (base ; ¹H, CDCl₃) : 1.69 (m ; 2H), 1.82 (m ; 2H), 1.95 (m ; 2H), 2.10 (m ; 2H), 3.31 (m ; 2H, H1 et H5), 3.65 (s ; 2H, N-CH₂-Ph), 4.42 (m ; 1H, H3), 4.57 (s ; 3H, OCH₃), 6.80 (d, J = 8.2Hz ; 1H, NH), 7.27 (m ; 1H), 7.32 (m ; 3H), 7.41 (d, J = 7.3Hz ; 1H), 7.80 (dd, J = 8.5, 0.7Hz ; 1H), 8.57 (d, J = 4.0Hz ; 1H)

| | | |
|---|---|---|
| Analyse | C₂₃ H₂₅ N₃ O₃ | Masse = 391.47 |
| | C₂₃ H₂₅ N₃ O₃, 2 HCl | Masse = 464.39 |
| | C₂₃ H₂₅ N₃ O₃, 2 HCl, 0.4 H₂O | Masse = 471.60 |

| | % C | %H | %N | % H₂O |
|---|---|---|---|---|
| calc. | 58.58 | 6.05 | 8.91 | 1.5 |
| trouv. | 58.89 | 6.05 | 8.24 | 1.5 |

SM (DCI, NH₃): 392 (M⁺ +1 ; 100%)

### Exemple 5:

### 3-Méthoxyfuro[2,3-c]pyridine-2-carboxylic acid (8-benzyl-8-azabicyclo[3.2.1]oct-3β-yl)amide fumarate

### a) 3-Hydroxyfuro[2,3-c]pyridine-2-carboxylic acid éthyl ester

Ce composé est préparé selon J.Hétérocycl.Chem. 1966, **3**, 252, et J.Hétérocycl.Chem. 1986, **23**, 549.
P_{F} = 170°C
RMN (¹H, CDCl₃) : 1.48 (t, J = 6.8Hz ; 3H), 4.52 (q, J = 6.8Hz ; 3H), 7.70 (d, J = 5.0Hz ; 1H), 8.52 (d, J = 5.0Hz ; 1H), 8.94 (s ; 1H)

### b) 3-Méthoxyfuro[2,3-c]pyridine-2-carboxylic acid éthyl ester

La méthylation de l'hydroxy se fait selon le mode opératoire de l'exemple 1 a).
RMN (¹H, CDCl₃) : 1.45 (t, J = 6.8Hz ; 3H), 4.29 (s ; 3H), 4.48 (q, J = 6.8Hz ; 2H), 7.73 (d, J = 5.2Hz ; 1H), 8.48 (d, J = 5.2Hz ; 1H), 8.96 (s ; 1H)

### c) 3-Méthoxyfuro[2,3-c]pyridine-2-carboxylic acid

Ce composé est préparé selon le mode opératoire de l'exemple 1 b).
PF = 260°C
RMN (¹H, DMSO-d₆) : 4.23 (s ; 3H), 8.01 (d, J = 5.4Hz ; 1H), 8.46 (d, J = 5.4Hz ; 1H), 9.05 (s ; 1H)

### d) 3-Méthoxyfuro[2,3-c]pyridine-2-carboxylic acid (8-benzyl-8-azabicyclo[3.2.1]oct-3β-yl)amide fumarate

Ce composé est préparé selon le mode opératoire de l'exemple 1 c).
PF= 101°C
IR : 3377, 2976, 1706, 1655
RMN (base ; ¹H, CDCl₃) : 1.66 (m ; 2H), 1.77 (m ; 2H), 1.94 (m ; 2H), 2.11 (m ; 2H), 3.29 (m ; 2H, H1 et H5), 3.56 (s ; 2H, N-CH₂-Ph), 4.29 (s ; 3H, OCH₃), 4.43 (m ; 1H, H3), 6.67 (d, J = 8.3Hz ; 1H, NH), 7.26 (m ; 1H), 7.33 (t, J = 7.3Hz ; 2H), 7.39 (d, J = 7.3Hz ; 2H), 7.68 (d ; J = 5.3Hz ; 1H), 8.45 (d, J = 5.3Hz ; 1H), 8.93 (s ; 1H)

| | | |
|---|---|---|
| Analyse | C₂₃ H₂₅ N₃ O₃ | Masse = 391.47 |
| | C₂₃ H₂₅ N₃ O₃, 1.5 C₄ H₄ O₄ | Masse = 565.58 |
| | C₂₃ H₂₅ N₃ O₃, 1.5 C₄ H₄ O₄, 1.4 H₂O | Masse = 590.81 |

| | % C | % H | % N | % H₂O |
|---|---|---|---|---|
| calc. | 58.96 | 5.76 | 7.11 | 4.3 |
| trouv. | 59.09 | 5.76 | 7.44 | 4.6 |

SM (ESI) : 393 (M+ +1), 392 (M+, 100%)

### Exemple 6:

### 3-Méthoxyfuro[2,3-b]pyridine-2-carboxylic acid (8-benzyl-8-azabicyclo[3.2.1]oct-3β-yl)amide fumarate

### a) 3-Hydroxyfuro[2,3-b]pyridine-2-carboxylic acid éthyl ester

Ce composé est préparé selon J.Hétérocycl.Chem. 1986, **23**, 1465.
RMN (¹H, CDCl3) : 1.46 (t, J = 7.2Hz ; 3H), 4.49 (q, J = 7.2Hz ; 2H), 7.31 (dd, J = 7.7, 4.8Hz ; 1H), 8.12 (dd, J = 7.2, 1.6Hz ; 1H), 8.28 (large ; 1H), 8.54 (dd, J = 4.8, 1.6Hz; 1H)

### b) 3-Méthoxyfuro[2,3-b]pyridine-2-carboxylic acid éthyl ester

La méthylation de l'hydroxy se fait selon le mode opératoire de l'exemple 1 a).
RMN (¹H, CDCl3) : 1.44 (t, J = 6.8Hz ; 3H), 3.90 (s ; 3H), 4.49 (q, J = 7.2Hz ; 2H), 6.92 (dd, J = 7.3, 5.0Hz ; 1H), 8.14 (dd, J = 7.3, 1.3Hz ; 1H), 8.29 (dd, J = 5.0, 1.3Hz ; 1H)

### c) 3-Méthoxyfuro[2,3-b]pyridine-2-carboxylic acid

Ce composé est préparé selon le mode opératoire de l'exemple 1 b).
PF = 260°C
RMN (¹H, CDCl3): 4.24 (s ; 3H), 7.45 (dd, J = 7.4, 5.1Hz ; 1H), 8.52 (m ; 2H)

### d) 3-Méthoxyfuro[2,3-b]pyridine-2-carboxylic acid (8-benzyl-8-azabicyclo[3.2.1]oct-3β-yl)amide fumarate

Ce composé est préparé selon le mode opératoire de l'exemple 1 c).
PF = 199°C
IR : 3395, 1665, 1596
RMN (base ; ¹H, CDCl₃) : 1.66 (m ; 2H), 1.78 (m ; 2H), 1.91 (m ; 2H), 2.07 (m ; 2H), 3.28 (m ; 2H, H1 et H5), 3.56 (s ; 2H, N-CH₂-Ph), 4.29 (s ; 3H, OCH₃), 4.39 (m ; 1H, H3), 6.56 (d, J = 8.3Hz ; 1H, NH), 7.26 (m ; 2H), 7.33 (t, J = 7.3Hz ; 2H), 7.40 (d, J = 7.3Hz ; 2H), 8.11 (dd, J = 7.8, 1.6Hz ; 1H), 8.45 (dd, J = 4.8, 1.6Hz; 1H)

| | | |
|---|---|---|
| Analyse | C₂₃ H₂₅ N₃ O₃ | Masse = 391.47 |
| | C₂₃ H₂₅ N₃ O₃, C₄ H₄ O₄ | Masse = 507.54 |
| | C₂₃ H₂₅ N₃ O₃, C₄ H₄ O₄, 0.2 H₂O | Masse = 511.14 |

| | %C | % H | %N | %H2O |
|---|---|---|---|---|
| calc. | 63.45 | 5.80 | 8.22 | 0.7 |
| trouv. | 63.40 | 5.84 | 8.22 | 0.5 |

SM (ESI) : 392 (M+ +1 ; 100%)

### Exemple 7:

### 3-Méthoxythièno[3,2-c]pyridine-2-carboxylic acid (8-benzyl-8-azabicylo[3.2.1]oct-3β-yl)amide fumarate

### a) 4-Chloropyridine-3-carbaldéhyde

Ce composé est préparé selon J.Hétérocycl.Chem. 1988, **25**, 81.

On additionne 35.8 ml de n-BuLi (1.6 M dans hexane ; 57 mmol, 1éq) à une solution de 7.5 ml de N,N-diisopropylamine dans 100 ml de tétrahydrofuranne à -60°C sous azote. Ensuite, on laisse agiter à 0°C pendant 45 min avant de refroidir à -90°C. A cette solution on introduit goutte à goutte une solution de 6.5 g de 4-chloropyridine (57 mmol, 1éq) dans 25 ml de tétrahydrofuranne de telle sorte que la température reste entre -70 et -60°C. On laisse agiter à -70°C pendant 3 h. Ensuite, on additionne une solution de 5.1 ml de l'éthylformate (63 mmol, 1.1éq) dans 10 ml de tétrahydrofuranne. On laisse agiter 1h à -65 / -70°C, ensuite, on laisse revenir le mélange réactionnel à -10°C et on hydrolyse par l'eau. On sépare la phase organique et on extrait la phase aqueuse 3 fois par l'acétate d'éthyle. On sèche les phases organiques réunies sur Na₂SO₄ et on évapore le solvant.

On obtient 5.91 g (73%) de produit formylé.
RMN (¹H, CDCl₃/DMSO-d₆) : 7.46 (d, J = 5.6Hz ; 1H), 8.69 (d, J = 5.6Hz ; 1H), 9.05 (s ; 1H), 10.51 (s ; 1H)

### b) 4-Chloronicotinic acid

On chauffe à reflux pendant 3 h un mélange des 5.9 g du produit du stade précédent a), (42 mmol, 1éq) avec 6.6 g de permanganate de potassium (42 mmole, 1éq) dans 200 ml de l'eau. Ensuite, on filtre à chaud et on lave le résidu par 500 ml d'eau chaude. On concentre le filtrat réuni jusqu'à 50 ml, on ajuste le pH à 3 par l'HCl 1N et on reconcentre la solution. On met cette solution à 4°C pour la cristallisation du produit qu'on filtre et sèche.

On obtient 3.71 g (56%) de l'acide recherché.
RMN (¹H, CDCl₃/DMSO-d₆) : 7.67 (d, J = 4.8Hz ; 1H), 8.66 (d, J = 4.8Hz ; 1H), 8.86 (s ; 1H), 14.23 (large ; 2H)

### c) 4-Chloronicotinic acid éthyl ester (voir J.Org.Chem. 1996, 26, 2257).

On additionne une solution de 1.5 g de l'hydroxyde de sodium (38 mmol, 1.6 éq) dans 8 ml d'eau à une solution de 3.71 g du produit du stade précédent b) (23 mmol, 1 éq) dans 50 ml de l'hexaméthylphosphorotriamide à 0°C. Ensuite, on introduit goutte à goutte pendant 1 h 7.5 ml de l'iodométhane (94 mmol, 4 éq). Après, on laisse revenir à température ambiante et on laisse agiter pendant 18 h. On dilue par 400 ml d'eau et on extrait 4 fois par de l'éther éthylique. On lave la phase organique soigneusement par l'eau avant de sécher sur Na₂SO₄ et d'évaporer le solvant.
On obtient 810 mg (18%) de l'ester recherché.
RMN (¹H, CDCl₃) : 1.44 (t, J = 7.2Hz ; 3H), 4.46 (q, J = 7.2Hz ; 1H), 7.58 (d, J = 5.6Hz ; 1H), 8.65 (d, J = 5.6Hz ; 1H), 9.08 (s ; 1H)

### d) 3-Hydroxythièno[3,2-c]pyridine-2-carboxylic acid méthyl ester

On chauffe à reflux pendant 48 h un mélange de 1.46 g du produit du stade précédent c) (7.8 mmol, 1éq) avec 0.7 ml de méthyl thioglycolate (7.8 mmol, 1 éq) dans 100 ml d'acétonitrile en présence 1.6 g de carbonate de potassium (11.8 mmol, 1.5 éq). Ensuite, on laisse revenir à température ambiante et on évapore le solvant. On dissout le résidu dans l'eau et on additionne de l'acide acétique jusqu'au pH 4. Le précipité formé est filtré, lavé par l'eau et séché.

On obtient 1.05 g (64%) du produit.
P_{F} = 144°C
RMN (¹H, CDCl₃) : 3.98 (s ; 3H), 7.68 (d, J = 5.7Hz ; 1H), 8.59 (d, J = 5.7Hz ; 1H), 9.22 (s ; 1H), 10.14 (large ; 1H)

### e) 3-Méthoxythièno[3,2-c]pyridine-2-carboxylic acid méthyl ester

La méthylation de l'hydroxy se fait selon le mode opératoire de l'exemple 1 a).
RMN (¹H, CDCl₃) : 3.94 (s ; 3H), 4.24 (s ; 3H), 7.67 (d, J = 5.5Hz ; 1H), 8.56 (d, J = 5.5Hz ; 1H), 9.17 (s ; 1H)

### f) 3-Méthoxythièno[3,2-c]pyridine-2-carboxylic acid

Ce composé est préparé selon le mode opératoire de l'exemple 1 b).
RMN (¹H , DMSO-d6) : 4.45 (s ; 3H), 8.04 (d, J = 5.6Hz ; 1H), 8.57 (d, J = 5.6Hz ; 1H), 9.13 (s ; 1H), 13.61 (large ; 1H)

### g) 3-Méthoxythièno[3,2-c]pyridine-2-carboxylic acid (8-benzyl-8-azabicylo[3.2.1]oct-3β-yl)amide fumarate

Ce composé est préparé selon le mode opératoire de l'exemple 1 c).
PF= 201°C
IR : 2372, 1702, 1644
RMN (base ; ¹H, CDCl₃) : 1.73 (m ; 2H), 1.80 (m ; 2H), 1.96 (m ; 2H), 2.11 (m ; 2H), 3.33 (m ; 2H, H1 et H5), 3.59 (s ; 2H, N-CH₂-Ph), 4.12 (s ; 3H, OCH₃), 4.40 (m ; 1H, H3), 7.28 (d, J = 7.2Hz ; 1H), 7.35 (t, J = 7.2Hz ; 2H), 7.41 (d, J = 7.2Hz ; 2H), 7.52 (d, J = 5.6Hz ; 1H), 8.51 (d, J = 5.6Hz ; 1H), 9.16 (s ; 1H)

| | | |
|---|---|---|
| Analyse | C₂₃ H₂₅ N₃ O₂ S | Masse = 407.54 |
| | C₂₃ H₂₅ N₃ O₂ S, C₄ H₄ O₄ | Masse = 523.61 |
| | C₂₃ H₂₅ N₃ O₂ S, 1.7 C₄ H₄ O₄, 0.23 H₂O | Masse = 609.09 |

| | %C | %H | %N | %H₂O |
|---|---|---|---|---|
| calc. | 58.76 | 5.37 | 6.90 | 0.7 |
| trouv. | 58.53 | 5.48 | 7.21 | 0.7 |

SM (ESI) : 430 (M⁺ +Na), 408 (M+, 100%)

### Exemple 8:

### 3-Méthoxythièno[2,3-b]pyridine-2-carboxylic acid [8-(4-chlorobenzyl)-8-azabicyclo[3.2.1]oct-3β-yl] amide chlorhydrate

### a) t-Butyl-N-[8-(phénylméthyl)-8-azabicyclo[3.2.1]oct-3β-yl]carbamate

On introduit à température ordinaire goutte à goutte une solution de 2.72 g du di-*ter*-butyldicarbonate (12.48 mmol, 1.2 éq) dans 10 ml de dichlorométhane à une solution de 2.25 g du 8-(phénylméthyl)-8-azabicyclo[3.2.1]octane-3β-amine (10.40 mmol, 1éq), préparée selon Eur.J.Med.Chem. 1984, **19**, 105, dans 20 ml de dichlorométhane en présence de 1.6 ml de triéthylamine (11.44 mmole, 1.1 éq). On laisse agiter à température ambiante jusqu'à la consommation totale des produits de départ. On lave trois fois par l'eau, on sèche et on évapore sous vide.

On obtient 1.6 g (49%) du produit après purification par filtration sur silice suivi d'une recristallisation dans l'éther isopropylique.
PF = 172-173°C
RMN (¹H, CDCl₃): 1.43 (s; 9H), 1.48 (m; 2H), 1.65-1.85 (m; 4H), 2.02 (m; 2H), 3.19 (m; 2H, H1 et H5), 3.52 (s; 2H, N-CH₂-Ph), 3.81 (m; 1H, H3), 4.30 (m; 1H, NH-carbamate), 7.19-7.38 (m; 5H)

### b) ter-Butyl-N-(8-azabicyclo[3.2.1]oct-3β-yl)carbamate

On hydrogène sous pression atmosphérique 1.6 g de *ter*-butyl-N-[8-(phénylméthyl)-8-azabicyclo[3.2.1]oct-3β-yl]carbamate (5.05 mmol, 1 éq) dissous dans 80 ml de méthanol à 45-50°C en présence du 0.1 g du charbon palladié à 10% pendant 90 min. On filtre le catalyseur, on rince par le méthanol et on évapore sous vide.

On obtient 1.1g (97%) du produit amine secondaire.
PF = 123°C
RMN (¹H, CDCl₃): 1.30 (m; 2H), 1.41 (s; 9H), 1.74 (m; 4H), 1.88 (m; 2H), 3.52 (m; 2H, H1 et H5), 3.78 (m; 1H, H3), 4.34 (m; 1H; NH-carbamate)

### c) ter-Butyl-[8-(4-chlorophénylméthyl)-8-azabicyclo[3.2.1]oct-3β-yl]carbamate

On chauffe à reflux pendant 15 h un mélange de 0.5 g du *ter*-butyl-N-(8-azabicyclo[3.2.1]oct-3β-yl]carbamate (2.43 mmol, 1éq) et de 0.4 g du chlorure de 4-chlorobenzyl (2.43 mmol, 1.1 éq) dans 30 ml de méthyléthylcétone en présence de 1.45 g du carbonate de césium (4.4 mmol, 2éq) et 0.2 g de l'iodure de potassium. On filtre l'insoluble, on rince par le solvant et on évapore sous vide. On reprend dans le dichlorométhane, on lave deux fois par l'eau et on évapore sous vide.

On obtient 0.45 g (58%)du produit d'alkylation après recristallisation dans le dichlorométhane / éther de pétrole.
PF = 131-132°C

### d) 8-(4-Chlorophénylméthyl)-8-azabicyclo[3.2.1]octane-3β-amine

On additionne goutte à goutte à température ordinaire une solution de 3 ml d'acide trifluoroacétique dans 3 ml du dichlorométhane à une solution de 0.45 g du *ter*-butyl-N-[8-(4-chlorophénylméthyl)-8-azabi-cyclo[3.2.1]oct-3β-yl]carbamate (1.28 mmol, 1 éq) dans 12 ml de dichlorométhane. Après 4h, on évapore sous vide, on reprend par la soude 1N et on extrait deux fois par le dichlorométhane. On lave par l'eau, on sèche et on évapore sous vide.

On obtient 0.32 g du produit avec un rendement quantitatif.
RMN (¹H, CDCl₃): 1.47-1.73 (m; 6H), 1.96 (m; 2H), 2.93 (m, J = 5.5Hz; 1H), 3.13 (t, J = 3.3Hz; 2H), 3.49 (s; 2H), 7.26 (m; 4H)

### e) 3-Méthoxythièno[2,3-b]pyridine-2-carboxylic acid [8-(4-chlorobenzyl)-8-azabicyclo[3.2.1]oct-3β-yl] amide chlorhydrate

Ce composé est préparé selon le mode opératoire de l'exemple 1 c).
PF = 261°C
IR : 3356, 1650, 1541
RMN (base ; ¹H, CDCl₃) : 1.63 (m ; 2H), 1.79 (m ; 2H), 1.97 (m ; 2H), 2.07 (m ; 2H), 3.26 (m ; 2H, H1 et H5), 3.51 (s ; 2H, N-CH₂-aryl), 4.10 (s ; 3H, OCH₃), 4.38 (m ; 1H, H3), 7.26-7.36 (m ; 5H), 8.09 (dd, J = 8.3, 1.4Hz ; 1H), 8.66 (dd, J = 4.6, 1.4Hz ; 1H)

| | | |
|---|---|---|
| Analyse | C₂₃ H₂₄ Cl N₃ O₂ S | Masse = 441.98 |
| | C₂₃ H₂₄ Cl N₃ O₂ S, HCl | Masse = 478.44 |

| | %C | %H | %N |
|---|---|---|---|
| calc. | 57.74 | 5.27 | 8.78 |
| trouv. | 57.92 | 5.26 | 8.72 |

SM (ESI) : 444 (M+ +2), 442 (M+ ; 100%)

### Exemple 9:

### 3-Méthoxythièno[2,3-b]pyridine-2-carboxylic acid [8-(4-méthoxybenzyl)-8-azabicyclo[3.2.1]oct-3β-yl] amide chlorhydrate

### a) ter-Butyl-N-[8-(4-méthoxyphénylméthyl)-8-azabicyclo[3.2.1]oct-3β-yl]carbamate

On chauffe à reflux pendant 20 h un mélange de 0.7 g du 4-*ter*-butyl-N-(8-azabicyclo[3.2.1]oct-3β-yl)carbamate (3.0 mmol, 1 éq), obtenu au stade b) de l'exemple 8, avec 0.45 ml du 4-méthoxybenzylchoride (3.3 mmol, 1.1 éq) dans 50 ml d'acétonitrile en présence de 2.0 g du carbonate de césium (6.0 mmol, 2 éq) et 0.25 g d'iodure de potassium. On filtre l'insoluble, on rince par le solvant et on évapore sous vide. On reprend dans le dichlorométhane, on lave deux fois par l'eau, on sèche et on évapore sous vide.

On obtient 0.8 g (77 %) du produit d'alkylation.
PF = 115°C
RMN (¹H, CDCl₃): 1.41-1.55 (m; 2H), 1.43 (s; 9H, t-butyl), 1.65-1.79 (m; 4H), 1.98-2.04 (m; 2H), 3.19 (m; 2H, H1 et H5), 3.46 (s; 2H, N-CH₂-Ar), 3.80 (m; 4H, OCH₃ et H3), 4.30 (m; 1H, NH-carbamate), 6.86 (d, J = 8.6Hz; 2H), 7.27 (d, J = 8.6Hz; 2H)

### b) 8-(4-Méthoxyphénylméthyl)-8-azabicyclo[3.2.1]octane-3β-amine

On additionne goutte à goutte à température ordinaire 3 ml d'acide trifluoroacétique à une solution de 0.80 g du *ter*-butyl-N-[8-(4-méthoxyphénylméthyl)-8-azabicyclo[3.2.1]oct-3β-yl]carbamate (2.3 mmol, 1éq), obtenu au stade précédent, dans 20 ml de dichlorométhane. Après 3 h, on évapore sous vide, on reprend par la soude 1N et on extrait deux fois par le dichlorométhane. On lave par l'eau, on sèche et on évapore sous vide.

On obtient 0.55g (96.5%) du produit.
PF < 50°C
RMN (¹H, DMSO-d₆): 1.29 (m; 2H), 1.42-1.55 (m; 4H), 1.88 (m; 2H), 2.78 (m; 1H, H3), 3.02 (m; 2H, H1 et H5), 3.40 (s; 2H, N-CH₂-aryl), 3.71 (s; OCH₃), 6.84 (d, J = 8.7Hz; 2H), 7.22 (d, J = 8.7Hz; 2H)

### c) 3-Méthoxythièno[2,3-b]pyridine-2-carboxylic acid [8-(4-méthoxybenzyl)-8-azabicyclo[3.2.1]oct-3β-yl] amide chlorhydrate

Ce composé est préparé selon le mode opératoire de l'exemple 1 c), mais avec les réactifs correspondants.
PF = 231°C
IR : 3397, 1651
RMN (base ; ¹H, CDCl₃) : 1.67 (m ; 2H), 1.79 (m ; 2H), 1.95 (m ; 2H), 2.10 (m ; 2H), 3.31 (m ; 2H, H1 et H5), 3.63 (s ; 2H, N-CH₂-aryl), 3.81 (s ; 3H, OCH₃), 4.09 (s ; 3H, OCH₃), 4.40 (m ; 1H, H3), 6.87 (d, J = 8.6Hz, 2H), 7.32 (m ; 3H), 8.08 (dd, J = 8.1, 1.4Hz ; 1H), 8.65 (dd, J = 4.5, 1.4Hz ; 1H)

| | | |
|---|---|---|
| Analyse | C₂₄ H₂₇ N₃ O₃ S | Masse = 437.56 |
| | C₂₄ H₂₇ N₃ O₃ S, 0.5 HCl | Masse = 455.79 |
| | C₂₄ H₂₇ N₃ O₃ S, 0.5 HCl, 0.1 H₂O | Masse = 457.79 |

| | %C | %H | %N | % H₂O |
|---|---|---|---|---|
| calc. | 63.00 | 6.10 | 9.18 | 0.4 |
| trouv. | 63.00 | 6.15 | 9.11 | 0.2 |

SM (ESI) : 438 (M+ + 1 ; 100%)

### Exemple 10:

### 3-Méthoxythièno[2,3-b]pyridine-2-carboxylic acid [8-cyclohexylméthyl-8-azabicyclo[3.2.1]oct-3β-yl] amide fumarate

### a) ter-Butyl-N-[8-(cyclohexylméthyl)-8-azabicyclo[3.2.1]oct-3β-yl]carbamate

On chauffe à reflux pendant 20 h un mélange de 0.7 g du *ter*-butyl-N-(8-azabicyclo[3.2.1]oct-3β-yl)carbamate (3.0 mmol, 1 éq), obtenu au stade b) de l'exemple 8, avec 0.46 ml du bromométhylcyclohexane (3.3 mmol, 1éq) dans 50 ml de méthyléthylcétone en présence de 2.0 g du carbonate de césium (6.0 mmol, 1éq) et 0.5 g de l'iodure de potassium (3.0 mmol, 1éq). On filtre l'insoluble, on rince par le solvant et on évapore sous vide. On reprend dans le dichlorométhane, on lave deux fois par l'eau, on sèche et on évapore sous vide. On obtient 0.97 g du produit avec un rendement quantitatif.
PF = 139-140°C
RMN (¹H, CDCl₃): 0.85 (m; 2H), 1.04-13.5 (m; 4H), 1.40 (s; 9H), 1.41-1.89 (m; 13H), 2.10 (d, J = 6.9 Hz; 2H, N-CH₂-cyclohexyl), 3.12 (m; 2H, H1 et H5), 3.74 (m; 1H, H3), 4.26 (m; 1H, NH-carbamate)

### b) 8-(Cyclohexylméthyl)-8-azabicyclo[3.2.1]octane-3β-amine

On additionne goutte à goutte à température ambiante 3 ml de l'acide trifluoroacétique à une solution de 0.97 g du *ter*-butyl-N-[8-(cyclohexylméthyl)-8-azabicyclo[3.2.1]oct-3β-yl]carbamate (3.0 mmol, 1éq) dans 15 ml de dichlorométhane. Après 2 h, on évapore sous vide, on reprend par la soude 1N et on extrait deux fois par le dichlorométhane. On lave par l'eau, on sèche et on évapore sous vide. On obtient 0.65g (97%) du produit de déprotection.
RMN (¹H, CDCl₃): 0.7-1.87 (m; 21H), 2.15 (d, J = 9.9Hz; 2H, N-CH₂-cyclohexyl), 2.85 (m, J = 5.6Hz; 1H, H3), 3.10 (t, J = 3.2Hz; 2H, H1 et H5)

### c) 3-Méthoxythièno[2,3-b]pyridine-2-carboxylic acid [8-cyclohexylméthyl-8-azabicyclo[3.2.1]oct-3β-yl] amide fumarate

Ce composé est préparé selon le mode opératoire de l'exemple 1 c).
PF = 245°C
IR : 3371, 1649, 1534
RMN (base ; ¹H, CDCl₃) : 0.91 (m ; 2H), 1.12-1.31 (m ; 4H), 1.66-1.74 (m ; 4H), 1.84 (m ; 2H), 1.96 (m ; 4H), 2.20 (m ; 2H), 3.29 (m ; 2H, H1 et H5), 4.10 (s ; 3H, OCH₃), 4.38 (m ; 1H, H3), 7.33 (dd, J = 8.2, 4.6hz ; 1H), 8.08 (dd, J = 8.2, 1.3Hz ; 1H), 8.65 (dd, J = 4.6, 1.3Hz ; 1H)

| | | |
|---|---|---|
| Analyse | C₂₃ H₃₁ N₃ O₂ S | Masse = 413.59 |
| | C₂₃ H₃₁ N₃ O₂ S, C₄ H₄ O₄ | Masse = 529.66 |

| | %C | %H | %N |
|---|---|---|---|
| calc. | 61.23 | 6.66 | 7.93 |
| trouv. | 61.31 | 6.75 | 7.96 |

SM (ESI) : 414.3 (M+ ; 100%)

### Exemple 11:

### 3-Méthoxythièno[2,3-b]pyridine-2-carboxylic acid [8-(4-fluorobenzyl)-8-azabicyclo[3.2.1]oct-3β-yl] amide chlorhydrate

En utilisant les mêmes matières premières que pour l'exemple 8, mais avec les réactifs correspondants, les intermédiaires suivants sont obtenus:

### a) t-Butyl-N-[8-(4-fluorobenzyl)-8-azabicyclo[3.2.1]oct-3β-yl]carbamate

RMN (¹H, CDCl3): 1.43 (s ; 9H), 1.46 (m ; 2H), 1.68 (m ; 2H), 1.82 (m ; 2H), 2.00 (m ; 2H), 3.17 (m ; 2H), 3.48 (s ; 2H), 3.79 (m ; 1H, H3), 4.30 (m ; 1H, NH-carbamate), 6.97 (d, J = 8.7Hz ; 1H), 6.99 (d, J = 8.7Hz ; 1H), 7.30 (dd, J = 8.7, 2.7Hz ; 2H)

### b) 8-(4-Fluorobenzyl)-8-azabicyclo[3.2.1]octane-3β-amine

RMN (¹H, DMSO-d6) : 1.32 (m ; 2H), 1.49-1.57 (m ; 4H), 1.90 (m ; 2H), 2.79 (septett, J = 5.7Hz ; 3H), 3.04 (m ; 2H), 3.47 (s ; 2H), 7.10 (d, J = 8.4Hz ; 1H), 7.13 (d, J = 8.4Hz ; 1H), 7.36 (dd, J = 8.4, 5.9Hz ; 2H)

### c) 3-Méthoxythièno[2,3-b]pyridine-2-carboxylic acid [8-(4-fluorobenzyl)-8-azabicyclo[3.2.1]oct-3β-yl] amide chlorhydrate

Ce composé est obtenu selon le mode opératoire de l'exemple 1 c), mais avec les réactifs correspondants.
PF = 247°C
IR : 3371, 1649, 1536
RMN (base ; ¹H, CDCl₃) : 1.63 (m ; 2H), 1.79 (m ; 2H), 1.96 (m ; 2H), 2.07 (m ; 2H), 3.26 (m ; 2H, H1 et H5), 3.64 (s ; 2H, N-CH2-aryl), 4.09 (s ; 3H, OCH₃), 4.39 (m ; 1H, H3), 7.02 (m ; 2H), 7.35 (m ; 3H), 8.09 (dd, J = 8.1, 1.5Hz ; 1H), 8.65 (dd, J = 4.6, 1.5Hz ; 1H)

| | | |
|---|---|---|
| Analyse | C₂₃ H₂₄ F N₃ O₂ S | Masse = 425.53 |
| | C₂₃ H₂₄ F N₃ O₂ S, HCl | Masse = 461.99 |
| | C₂₃ H₂₄ F N₃ O₂ S, 0.84 HCl, 0.14 H₂O | Masse = 458.67 |

| | %C | %H | %N | %H₂O |
|---|---|---|---|---|
| calc. | 60.23 | 5.52 | 9.16 | 0.55 |
| trouv. | 60.22 | 5.47 | 9.14 | 0.6 |

SM (ESI) : 447.9 (M⁺ + H2O), 425.9 (M⁺, 100%)

### Exemple 12:

### 3-Méthoxythièno[2,3-b]pyridine-2-carboxylic acid [8-thiophèn-3-ylméthyl-8-azabicyclo[3.2.1]oct-3β-yl] amide chlorhydrate

### a) 3-Bromométhylthiophène

Ce composé est décrit dans: Org.Synth.Coll.Vol. IV, 1963, 921.

On chauffe à reflux pendant 2 h un mélange de 4.4 g du 3-méthylthiophène (44.8 mmol, 1éq) et 7.15 g du N-bromosuccinimide (40 mmol, 0.9 éq) en présence de 0.1 g du peroxyde de benzoyle dans 100 ml du tétrachlorométhane. On filtre l'insoluble, on évapore sous vide et on purifie par distillation sous vide. On obtient 1.2 g du produit bromé.
RMN (¹H, CDCl₃): 4.51 (s; 2H), 7.10 (d, J = 4.5Hz; 1H), 7.27 (d, J = 1.8Hz; 1H), 7.29 (dd, J = 4.5, 1.8Hz; 1H)

En utilisant les mêmes matières premières que pour l'exemple 8, mais avec les réactifs correspondants, les intermédiaires suivants sont obtenus:

### b) t-Butyl-N-[8-(3-thiénylméthyl)-8-azabicyclo[3.2.1]oct-3β-yl]carbamate

On obtient 0.9g du produit (63%) après cristallisation dans l'éther isopropylique / éther de pétrole.
PF= 138°C
RMN (¹H, CDCl₃): 1.45-1.56 (m; 2H), 1.44 (s; 9H, t-butyl), 1.60-1.85 (m; 2H), 1.99 (m; 2H), 3.22 (m; 2H, H1 et H5), 3.54 (s; 2H, N-CH₂-Ar), 3.78 (m; 1H, H3), 4.30 (m; 1H, NH-carbamate), 7.07-7.12 (m; 2H), 7.24-7.29 (m; 1H)

### c) 8-(3-Thiénylméthyl)-8-azabicyclo[3.2.1]oct-3β-amine

On obtient 0.50g de l'amine (86%).
RMN (¹H, DMSO-d₆) : 1.25-1.36 (m; 4H), 1.43-1.59 (m; 4H), 1.85-1.91 (m; 2H), 2.78 (m; 1H, H3), 3.07 (t, J = 3.0Hz; 2H, H1 et H5), 3.48 (s; 2H, N-CH₂-Ar), 7.06 (d, J = 4.9Hz; 1H), 7.26 (d, J = 2.8Hz; 1H), 7.44 (dd, J = 4.9, 2.8Hz; 1H)

### d) 3-Méthoxythièno[2,3-b]pyridine-2-carboxylic acid [8-thiophèn-3-ylméthyl-8-azabicyclo[3.2.1]oct-3β-yl] amide chlorhydrate

Ce composé est préparé selon le mode opératoire de l'exemple 1 c), mais avec les réactifs correspondants.
PF = 230°C
IR : 3385, 1641, 1534
RMN (base ; ¹H, CDCl₃) : 1.63 (m ; 2H), 1.78 (m ; 2H), 1.97 (m ; 2H), 2.08 (m ; 2H), 3.32 (m ; 2H, H1 et H5), 3.57 (s ; 2H, N-CH₂-aryl), 4.09 (s ; 3H, OCH₃), 4.39 (m ; 1H, H3), 7.14 (m ; 2H), 7.29-7.35 (m ; 3H), 8.08 (dd, J = 8.1, 1.5Hz ; 1H), 8.65 (dd, J = 4.7, 1.5Hz ; 1H)

| | | |
|---|---|---|
| Analyse | C₂₁ H₂₃ N₃ O₂ S₂ | Masse = 413.56 |
| | C₂₁ H₂₃ N₃ O₂ S₂, HCl | Masse = 450.02 |
| | C₂₁ H₂₃ N₃ O₂ S₂, 0.85 HCl, 0.24 H₂O | Masse = 448.77 |

| | %C | %H | %N | % H₂O |
|---|---|---|---|---|
| calc. | 56.20 | 5.46 | 9.36 | 0.9 |
| trouv. | 56.21 | 5.82 | 8.87 | 1.1 |

SM (ESI) : 436 (M⁺ + Na), 414 (M⁺ +1 ; 100%)

### Exemple 13:

### 3-Méthoxythièno[2,3-c]pyridine-2-carboxylic acid (8-benzyl-8-azabicyclo[3.2.1]oct-3β-yl]amide fumarate

### a) 3-Bromo-4-picoline

Ce réactif est décrit dans le Bull. Soc. Chim. Fr. 1976, 530.

On additionne sous bonne agitation, goutte à goutte 31 ml de 4-picoline (0.322 mol, 1éq) à 107 g du chlorure d'aluminium (0.805 mol, 2.5 éq) dans un tricol de 1 l équipé d'un réfrigérant sous azote. Ensuite, on introduit progressivement 9.9 ml du brome (0.193 mol, 0.6 éq) en gardant la température à 95-100°C. On laisse agiter à cette température pendant 18 h, on additionne 6.6 ml du brome (0.129 mol, 0.4 éq) et continue l'agitation pendant 4 h. Après, on verse le milieu réactionnel dans 1000 ml de glace, on additionne la soude jusqu'à la dissolution des sels inorganiques. On récupère la phase huileuse, on extrait la phase aqueuse 3 fois par l'éther éthylique et on lave la phase organique réunie avec une solution de bisulfite de sodium puis à l'eau. On sèche sur Na₂SO₄ et on évapore le solvant.

On obtient 10.68 g (19%) du produit bromé après purification par chromatographie flash sur silice (éluant: acétate d'éthyle / éther de pétrole = 1/1).
RMN (¹H, CDCl₃): 2.40 (s ; 3H), 7.17 (d, J = 4.9Hz ; 1H), 8.37 (d, J = 4.9Hz ; 1H), 8.64 (s ; 1H)

### b) 3-Bromoisonicotinic acid

On chauffe à reflux un mélange de 10.68 g du produit de stade précédent (62 mmol, 1éq) avec 29.4 g de permanganate de potassium (186 mmol, 3 éq) dans 100 ml d'eau. On filtre l'oxyde de manganèse formé à chaud, on lave le précipité par l'eau bouillante et on récupère le filtrat. On laisse revenir le filtrat à température ambiante, on extrait 3 fois par l'éther éthylique et on acidifie la phase aqueuse par l'HCl (pH 2-3). On concentre la solution jusqu'à 50 ml de volume et on la garde à 4°C pendant 2h. On filtre les cristaux formés, on les lave par l'eau et on sèche. On obtient 3.98 g du produit (32%).
PF = 240°C
RMN (¹H, DMSO-d₆) : 4.39 (large ; 1H), 7.70 (d, J = 4.9Hz ; 1H), 8.67 (d, H = 4.9Hz ; 1H), 8.88 (s ; 1H)

### c) 3-Bromoisonicotinic acid méthyl ester

On chauffe à reflux un mélange de 3.98 g de l'acide obtenu au stade précédent (20 mmol, 1éq) dans 50 ml du méthanol en présence de 4 ml de l'acide sulfurique concentré. Ensuite, on laisse revenir à température ambiante et on extrait 3 fois par l'acétate d'éthyle. On sèche la phase organique sur Na₂SO₄ et on évapore le solvant. On obtient 2.65g (62%) du produit estérifié.
RMN (¹H, CDCl₃) : 4.02 (s ; 3H), 7.64 (d, J = 4.9Hz ; 1H), 8.63 (d, J = 4.9Hz ; 1H), 8.88 (s ; 1H)

### d) 3-Hydroxythièno[2,3-c]pyridine-2-carboxylic acid méthyl ester

On chauffe à reflux pendant 18 h un mélange de 2.65 g du produit de stade précèdent (12 mmol, 1éq) avec 1.1 ml du méthyl thioglycolate (12 mmol, 1éq) dans 100 ml d'acétonitrile en présence de 2.54 g de carbonate de potassium (18 mmol, 1.5éq). Après, on laisse revenir à température ambiante, on évapore le solvant et on dissout le résidu dans l'eau. On additionne quelques ml d'acide acétique (pH 4) et on filtre le précipité formé. On le sèche sous vide. On obtient 1.51 g (59%) du produit cyclisé.
RMN (¹H, CDCl₃) : 4.00 (s ; 3H), 7.63 (d, J = 4.9Hz ; 1H), 8.58 (d, J = 4.9Hz ; 1H), 9.10 (s ; 1H), 10.02 (s ; 1H)

### e) 3-Méthoxythièno[2,3-c]pyridine-2-carboxylic acid méthyl ester

Ce composé est préparé selon le mode opératoire de l'exemple 1 a).
RMN (¹H, CDCl3) : 3.96 (s ; 3H), 4.18 (s ; 3H), 7.74 (d, J = 5.5Hz ; 1H), 8.56 (d, J = 5.5Hz ; 1H), 9.09 (s ; 1H)

### f) 3-Méthoxythièno[2,3-c]pyridine-2-carboxylic acid

Ce composé est préparé selon le mode opératoire de l'exemple 1 b).
RMN (¹H, CDCl3) : 4.10 (s ; 3H), 7.83 (d, J = 5.5Hz ; 1H), 8.55 (d, J = 5.5Hz ; 1H), 9.27 (s ; 1H), 13.72 (large ; 1H)

### g) 3-Méthoxythièno[2,3-c]pyridine-2-carboxylic acid (8-benzyl-8-azabicyclo[3.2.1]oct-3β-yl]amide fumarate

Ce composé est préparé selon le mode opératoire de l'exemple 1 c).
PF = 173°C
IR : 3372, 1697, 1646, 1534
RMN (base ; ¹H, CDCl₃) : 1.65 (m ; 2H), 1.78 (m ; 2H), 1.97 (m ; 2H), 2.11 (m ; 2H), 3.30 (m ; 2H, H1 et H5), 3.55 (m ; 2H, N-CH₂-Ph), 4.12 (s ; 3H, OCH₃), 4.39 (m ; 1H, H3), 7.26 (m ; 1H), 7.34 (m ; 2H), 7.39 (d, J = 7.2Hz ; 2H), 7.67 (d, J = 5.6Hz ; 1H), 8.53 (d, J = 5.6Hz ; 1H), 9.10 (s ; 1H)

| | | |
|---|---|---|
| Analyse | C₂₃ H₂₅ N₃ O₂ S | Masse = 407.54 |
| | C₂₃ H₂₅ N₃ O₂ S, C₄ H₄ O₄ | Masse = 523.61 |
| | C₂₃ H₂₅ N₃ O₂ S, 1.4 C₄ H₄ O₄, 0.1 H₂O | Masse = 571.75 |

| | %C | %H | %N | % H₂O |
|---|---|---|---|---|
| calc. | 60.10 | 5.43 | 7.35 | 0.3 |
| trouv. | 60.10 | 5.46 | 7.21 | 0.3 |

SM (ESI) : 430 (M⁺ + Na), 408 (M⁺ +1 ; 100%)

### Exemple 14:

### 3-Isopropoxythièno[2,3-b]pyridine-2-carboxylic acid (8-benzyl-8-azabicyclo[3.2.1]oct-3β-yl]amide fumarate

### a) 3-Isopropoxythièno[2,3-b]pyridine-2-carboxylic acid méthyl ester

On introduit 1.04 g du 3-hydroxythièno[2,3-*b*]pyridine-2-carboxylic acid méthyl ester (5.0mmole, 1éq), obtenu selon J.Hétérocycl.Chem. 1987, **24**, 85, à une solution de 2.8 g de *ter*-butylate de potassium (25 mmol, 5éq) dans 15 ml de DMSO à 0°C sous azote. Après 30 min, on additionne 2.35 ml de 2-bromopropane. Après 2 h d'agitation à température ambiante on chauffe à 100°C pendant 8 h. On laisse revenir à température ambiante, on verse le milieu réactionnel sur 200 ml d'eau et on extrait 2 fois par l'acétate d'éthyle. On lave la phase organique par de l'eau saturée de chlorure de sodium, on sèche sur Na₂SO₄ et on évapore le solvant.

On obtient 880 mg (70%) du produit de O-alkylation.
RMN (¹H, CDCl₃) : 1.43 (d, J = 6.0Hz ; 6H), 3.93 (s ; 3H), 4.86 (septett, J = 6.0Hz ; 1H), 7.30 (dd, J = 8.0, 4.4Hz ; 1H), 8.10 (d, J = 8.0, 1.4Hz ; 1H), 8.56 (d, J = 4.4, 1.4Hz ; 1H)

### b) 3-Isopropoxythièno[2,3-b]pyridine-2-carboxylic acid

Ce composé est préparé selon le mode opératoire de l'exemple 1 b).
RMN (¹H, DMSO-d₆) : 1.31 (d, J = 6.0Hz ; 6H), 4.83 (septett, J = 6.0Hz ; 1H), 7.52 (d, J = 8.1, 4.6Hz ; 1H), 8.26 (d, J = 8.1Hz ; 1H), 8.74 (d, J = 4.6Hz ; 1H), 13.43 (large ; 1H)

### c) 3-Isopropoxythièno[2,3-b]pyridine-2-carboxylic acid (8-benzyl-8-azabicyclo[3.2.1]oct-3β-yl]amide fumarate

Ce composé est préparé selon le mode opératoire de l'exemple 1 c).
PF = 184°C
IR : 3390, 1649, 1535
RMN (base ; ¹H, CDCl₃) : 1.44 (d, J = 6.4Hz ; 6H, CH-(CH₃)₂), 1.66 (m ; 2H), 1.78 (m ; 2H), 1.96 (m ; 2H), 2.09 (m ; 2H), 3.29 (m ; 2H, H1 et H5), 3.57 (s ; 2H, N-CH₂-Ph), 4.74 (septett, J = 6.4Hz ; 1H, CH-(CH₃)₂), 7.26 (m ; 1H), 7.31 (m ; 3H), 7.39 (d, J = 7.3Hz ; 1H), 8.01 (dd, J = 8.2, 1.5Hz ; 1H), 8.44 (dd, J = 4.6, 1.5Hz ; 1H)

| | | |
|---|---|---|
| Analyse | C₂₅ H₂₉ N₃ O₂ S | Masse = 435.59 |
| | C₂₅ H₂₉ N₃ O₂ S, C₄ H₄ O₄ | Masse = 551.67 |

| | %C | %H | %N |
|---|---|---|---|
| calc. | 63.14 | 6.03 | 7.62 |
| trouv. | 62.96 | 6.06 | 7.64 |

SM (ESI) : 437 (M⁺ +2), 436 (M⁺ +1 ; 100%)

### Exemple 15:

### 3-Éthoxythièno[2,3-b]pyridine-2-carboxylic acid (8-benzyl-8-azabicyclo[3.2.1]oct-3β-yl)amide fumarate

### a) 3-Éthoxythièno[2,3-b]pyridine-2-carboxylic acid méthyl ester

Ce composé est préparé selon le mode opératoire de l'exemple 14 a), mais avec le réactif correspondant, l'iodure d'éthyle.
RMN (¹H, CDCl₃) : 1.52 (t, J = 7.2Hz ; 3H), 3.93 (s ; 3H), 4.18 (q, J = 7.2Hz ; 2H), 7.28 (dd, J = 8.0, 4.5Hz ; 1H), 8.06 (dd, J = 8.0, 1.3Hz ; 1H), 8.57 (dd, J = 4.5, 1.3Hz ; 1H)

### b) 3-Éthoxythièno[2,3-b]pyridine-2-carboxylic acid

Ce composé est préparé selon le mode opératoire de l'exemple 1 b).
PF = 206°C
RMN (¹H, DMSO-d₆) : 1.37 (t, J = 6.8Hz ; 3H), 4.37 (q, J = 6.8Hz ; 2H), 7.53 (dd, J = 7.8, 4.6Hz ; 1H), 8.28 (d, J = 7.8Hz ; 1H), 8.74 (d, J = 4.6Hz ; 1H), 13.47 (large ; 1H)

### c) 3-Éthoxythièno[2,3-b]pyridine-2-carboxylic acid (8-benzyl-8-azabicyclo[3.2.1]oct-3β-yl)amide fumarate

Ce composé est préparé selon le mode opératoire de l'exemple 1 c).
PF = 170°C
IR : 3357, 1685, 1536
RMN (base ; ¹H, CDCl₃) : 1.51 (t, J = 7.2Hz ; 3H, CH₂-CH₃), 1.65 (m ; 2H), 1.78 (m ; 2H), 1.97 (m ; 2H), 2.10 (m ; 2H), 3.28 (m ; 2H, H1 et H5), 3.56 (s ; 2H, N-CH₂-Ph), 4.35 (q, J = 7.2Hz ; 2H, O-CH₂-CH₃), 4.38 (m ; 1H, H3), 7.26 (m ; 1H), 7.33 (m ; 3H), 7.39 (d, J = 7.2Hz ; 2H), 8.04 (dd, J = 6.7, 1.5Hz ; 1H), 8.64 (dd, J = 4.6, 1.5Hz ; 1H)

| | | |
|---|---|---|
| Analyse | C₂₄ H₂₇ N₃ O₂ S | Masse = 421.56 |
| | C₂₄ H₂₇ N₃ O₂ S, C₄ H₄ O₄ | Masse = 537.63 |
| | C₂₄ H₂₇ N₃ O₂ S, C₄ H₄ O₄, 1/3 H₂O | Masse = 543.58 |

| | %C | %H | %N | % H₂O |
|---|---|---|---|---|
| calc. | 61.87 | 5.87 | 7.73 | 1.1 |
| trouv. | 61.96 | 5.81 | 7.65 | 1.1 |

SM (ESI) : 422 (M⁺ +1 ; 100%)

### Exemple 16:

### 3-Méthoxyfuro[3,2-b]pyridine-2-carboxylic acid [8-(4-fluorobenzyl)-8-azabicyclo[3.2.1]oct-3β-yl]amide chlorhydrate

### a) 3-Méthoxyfuro[3,2-b]pyridine-2-carboxylic acid [8-azabicyclo[3.2.1]oct-3β-yl]amide

On additionne une solution de 1.49 g de formate d'ammonium (22 mmol, 4 éq) dans 6 ml d'eau à une solution de 2.15 g de 3-méthoxyfuro[3,2-*b*]pyridine-2-carboxylic acid [8-benzyl)-8-azabicyclo[3.2.1]oct-3β-yl]amide (5.49 mmol, 1 éq), obtenu à l'exemple 4 f), dans 50 ml de méthanol. On y ajoute 200 mg de Pd(OH)₂/C et on chauffe à reflux pendant 18 h. On laisse revenir à température ambiante, on filtre le catalyseur et on évapore le solvant. On reprend le résidu dans le dichlorométhane, on lave 2 fois par l'eau suivie d'une extraction par 3 fois 100 ml d'HCl 1N. On alcalinise l'extrait par la soude (pH 10) et on extrait 3 fois par le dichlorométhane. On sèche, on évapore le solvant et on purifie par chromatographie flash sur silice (éluant: dichlorméthane / methanol /ammoniaque = 95 / 4.5 / 0.5). On obtient 1.73 g (85%) du produit de débenzylation.
RMN (¹H,CDCl₃) : 1.61 (m ; 2H), 1.89 (m ; 2H), 2.05 (m ; 2H), 2.08 (m ; 2H), 2.40 (large ; 1H), 3.68 (m ; 2H), 4.44 (m ; 1H, H3), 4.56 (s ; 3H), 6.81 (d, J = 8.5Hz ; 1H, 7.32 (dd, J = 8.5, 4.6Hz ; 1H), 8.81 (dd, J = 8.5, 1.2Hz ; 1H), 8.57 (dd, J = 4.6, 1.2Hz ; 1H)

### b) 3-Méthoxyfuro[3,2-b]pyridine-2-carboxylic acid [8-(4-fluorobenzyl)-8-azabicyclo[3.2.1]oct-3b-yl]amide chlorhydrate

On chauffe à reflux pendant 18 h un mélange de 580 mg du produit de stade précédent (1.9 mmol, 1éq) avec 0.24 ml de bromure de 4-fluorobenzyl (1.9 mmol, 1 éq) dans 50 ml du méthyléthylcétone en présence de 270 mg du carbonate de potassium (1.9 mmol) et 50 mg de l'iodure de potassium. Après, on laisse revenir à température ambiante et on évapore le solvant. On reprend le résidu dans le dichlorométhane et on lave 3 fois par l'eau. On sèche la phase organique, on évapore le solvant et on purifié par chromatographie flash sur silice (éluant: dichlorométhane).

On obtient 370 mg du produit (47%) sous forme de base qui est ensuite salifiée par une quantité stoechiométrique d'une solution d'HCl dans l'isopropanol.
PF = 242°C
IR : 3367, 1670, 1540
RMN (base ; ¹H, CDCl₃) : 1.65 (m ; 2H), 1.83 (m ; 2H), 1.95 (m ; 2H), 2.04 (m ; 2H), 3.26 (m; 2H), 3.52 (s ; 2H, N-CH₂-Ph), 4.40 (m ; 1H, H3), 4.57 (s ; 3H, OCH₃), 6.77 (d, J = 8.3Hz ; 1H, NH), 7.01 (t, J = 8.6Hz ; 2H), 7.32 (dd, J = 8.4, 4.5Hz ; 1H), 7.36 (m ; 2H), 7.79 (d, J = 8.4Hz ; 1H), 8.57 (d, J = 4.5Hz ; 1H)

| | | |
|---|---|---|
| Analyse | C₂₃ H₂₄ F N₃ 03 | Masse = 409.46 |
| | C₂₃ H₂₄ F N₃ O₃, HCl | Masse = 445.92 |
| | C₂₃ H₂₄ F N₃ O₃, 1.1 HCl, 0.1 H₂O | Masse = 451.37 |

| | % C | % H | % N | % H2O |
|---|---|---|---|---|
| calc. | 61.20 | 5.65 | 9.31 | 0.4 |
| trouv. | 61.09 | 5.65 | 9.11 | 0.4 |

### Exemple 17:

### 3-Méthoxyfuro[3,2-b]pyridine-2-carboxylic acid [8-(4-chlorobenzyl)-8-azabicyclo[3.2.1]oct-3β-yl]amide chlorhydrate

Selon le mode opératoire de l'exemple 16, mais avec les réactifs correspondants ce dérivé est obtenu:
PF = 232°C
IR : 3392, 1675, 1540
RMN (base ; ¹H, CDCl₃) : 1.62 (m ; 2H), 1.81 (dd, J = 14.0, 6.0Hz ; 2H), 1.95 (m ; 2H), 2.06 (m ; 2H), 3.25 (m ; 2H), 3.52 (s ; 2H, N-CH₂-Ph), 4.39 (m ; 1H, H3), 4.57 (s ; 3H, OCH₃), 6.77 (d, J = 8.2Hz ; 1H, NH), 7.28-7.35 (m ; 5H), 7.79 (dd, J = 8.4, 1.0Hz ; 1H), 8.57 (dd, J = 4.5, 1.0Hz ; 1H)

| | | |
|---|---|---|
| Analyse | C₂₃ H₂₄ Cl N₃ O₃ | Masse = 425.92 |
| | C₂₃ H₂₄ Cl N₃ O₃, HCl | Masse = 462.38 |
| | C₂₃ H₂₄ Cl N₃ O₃, HCl, 0.13 H₂O | Masse = 464.72 |

| | % C | % H | %N | % H₂O |
|---|---|---|---|---|
| calc. | 59.45 | 5.35 | 8.86 | 0.5 |
| trouv. | 59.46 | 5.38 | 8.82 | 0.5 |

### Exemple 18:

### 3-Méthoxyfuro[3,2-b]pyridine-2-carboxylic acid (8-cyclohexylméthyl-8-azabicyclo[3.2.1]oct-3β-yl)amide chlorhydrate

Selon le mode opératoire de l'exemple 16, mais avec les réactifs correspondants ce dérivé est obtenu:
PF = 217°C
IR : 3291, 1659, 1537
RMN (base ; ¹H, CDCl₃) : 0.91 (m ; 2H), 1.12-1.35 (m ; 3H), 1.37 (m ; 1H), 1.62-1.85 (m ; 7H), 1.86 (m ; 2H), 1.89-1.97 (m ; 4H), 2.16 (d, J = 6.8Hz ; 2H), 3.23 (m ; 2H), 4.35 (m ; 1H, H3), 4.56 (s ; 3H, OCH₃), 6.76 (d, J = 8.2Hz ; 1H, NH), 7.33 (dd, J = 8.4, 4.5Hz ; 1H), 7.79 (d, J = 8.4Hz ; 1H), 8.56 (d, J = 4.5Hz ; 1H)

| | | |
|---|---|---|
| Analyse | C₂₃ H₃₁ N₃ O₃ | Masse = 397.52 |
| | C₂₃ H₃₁ N₃ O₃, HCl | Masse = 433.98 |
| | C₂₃ H₃₁ N₃ O₃, HCl, 0.17 H₂O | Masse = 437.02 |

| | %C | %H | %N | % H₂O |
|---|---|---|---|---|
| calc. | 63.21 | 7.46 | 9.61 | 0.7 |
| trouv. | 62.93 | 7.46 | 9.27 | 0.7 |

SM (ESI, 250°C) : 398 (M⁺ ; 100%)

### Exemple 19:

### 3-Méthoxythièno[2,3-b]pyridine-2-carboxylic acid [8-butyl-8-azabicyclo[3.2.1]oct-3β-yl] amide fumarate

Ce composé est préparé selon le mode opératoire de l'exemple 8, mais en utilisant les réactifs correspondants.
a) t-Butyl-N-[8-butyl-8-azabicyclo[3.2.1]oct-3β-yl]carbamate
   RMN (¹H, CDCl₃) : 0.91 (t, J = 7.6Hz ; 3H), 1.30 (p, J = 7.6Hz ; 2H), 1.43 (s ; 9H), 1.40-1.48 (m ; 4H), 1.63 (m ; 2H), 1.79 (m ; 2H), 1.92 (m ; 2H) 2.31 (d, J = 7.6Hz ; 1H), 2.33 (d, J = 7.6Hz ; 1H), 3.24 (m ; 2H), 3.78 (m ; 1H, H3), 4.28 (large ; 1H, NH-carbamate)
b) 8-Butyl-8-azabicyclo[3.2.1]octane-3β-amine
   RMN (¹H, DMSO-d6) : 0.87 (t, J = 7.2Hz ; 3H), 1.23-1.35 (m ; 6H), 1.43 (m ; 2H), 1.48 (m ; 2H), 1.77 (m ; 2H), 2.27 (t, J = 7.2Hz ; 2H), 2.75 (septett, J = 5.6Hz ; 1H, H3), 3.03 (m ; 2H)
c) 3-Méthoxythièno[2,3-*b*]pyridine-2-carboxylic acid [8-butyl-8-azabicyclo[3.2.1]oct-3β-yl] amide fumarate
   PF = 211°C
   IR : 3374, 1641, 1535
   RMN (sel ; ¹H, CD₃OD) : 1.02 (t, J = 7.6Hz ; 3H), 1.45 (sextett, J = 7.6Hz ; 2H), 1.74 (m ; 2H), 2.10-2.31 (m ; 6H), 2.33 (m ; 2H), 3.10 (m ; 2H), 4.02 (m ; 2H), 4.20 (m ; 2H), 4.46 (m ; 1H, H3), 6.69 (s ; 2H, acide fumarique), 7.50 (dd, J = 8.3, 4.7Hz ; 1H), 8.40 (dd, J = 8.3, 1.5Hz ; 1H), 8.65 (dd, J = 4.7, 1.5Hz ; 1H)

| | | |
|---|---|---|
| Analyse | C₂₀ H₂₇ N₃ O₂ S | Masse = 373.52 |
| | C₂₀ H27 N₃ O₂ S, C₄ H₄ O₄ | Masse = 489.60 |

| | %C | %H | %N |
|---|---|---|---|
| calc. | 58.88 | 6.38 | 8.58 |
| trouv. | 58.90 | 6.41 | 8.56 |

SM (ESI) : 374.2 (M⁺ , 100%)

### Exemple 20:

### 3-Méthoxyfuro[2,3-b]pyrazine-2-carboxylic acid (8-benzyl-8-azabicyclo[3.2.1]oct-3β-yl)amide chlorhydrate

### a) 3-Chloro-2-pyrazine carboxylate de méthyle.(J.Chem.Soc. Perkin Trans. I, 1996, 247)

Dans 20 ml d'acide chlorhydrique concentré, on introduit 3.06 g (20 mmol, 1 éq) de 3-amino-2-pyrazine carboxylate de méthyle, à 0°C. On y introduit alors, goutte à goutte, une solution de 1.52 g (22 mmlo, 1.1éq) de NaNO2 dans 15 ml d'eau, en limitant la température à <5°C, jusqu'à cessation du dégagement gazeux. La solution obtenue est filtrée et additionnée lentement à une solution d'acétate de sodium (20 g) dans 40 ml d'eau à 0°C. L'agitation est maintenue pendant 15 min, puis acidifiée par HCl concentré. L'extraction (4 fois 30 ml d'acétate d'éthyle) fournit après séchage et évaporation 1.35 g du composé chloré.
RMN (¹H, CDCl₃) : 4.05 (s ; 3H; OCH3), 8.54 (d, J = 2.4Hz ; 1H; H arom.), 8.60 (d, J = 2.4Hz; 1H; H arom.)

### b) 3-hydroxy-2-éthoxycarbonylfuro[2,3-b]pyrazine

On additionne goutte à goutte une solution de 17.4 ml (0.184 mmol, 2.8 éq) de glycolate d'éthyle dans 20 ml de DME, à une suspension 6.85 g (0.171 mol, 2.6 éq) de NaH (60% dans l'huile) à 0°C dans 200 ml de DME. Après avoir laissé en contact sous agitation pendant 15 min, on introduit alors 11.36 g du dérivé chloré du stade a) précédent, en solution dans 20 ml de DME. Après retour à température ordinaire, le milieu est chauffé à 80°C pendant 9h. Après évaporation et reprise dans l'eau, le milieu est acidifié avec l'acide acétique jusqu'à pH4, puis extrait par 5 fois 50 ml d'acétate d'éthyle. Après séchage sur Na2SO4 , filtration et évaporation, le résidu est trituré dans l'éther de pétrole, puis cristallisé, filtré et séché sous vide. On obtient 9.91 g (72%) de produit de cyclisation. PF = 160°C (dec).
RMN (¹H, CDCl₃): 1.48 (t; J = 7.2Hz; 3H; CH3 ester), 4.53 (q; J = 7.2Hz; 2H; CH2 ester), 8.41 (m; 1H; OH), 8.50 (d, J = 2.4Hz ; 1H; H arom.), 8.69 (d, J = 2.4Hz; 1H; H arom.)

### c) 3-Méthoxy-2-éthoxycarbonylfuro[2,3-b]pyrazine

Ce composé est obtenu selon le mode opératoire de l'exemple 1 a).
Rdt = 76%.
RMN (¹H, CDCl₃) : 1.44 (t; J = 7.2Hz; 3H; CH3 ester), 4.48 (q; J = 7.2Hz ; 2H; CH2 ester), 4.58 (s; 3H; OCH3), 8.46 (d, J = 2.4Hz ; 1H; H arom.), 8.64 (d, J = 2.4Hz; 1H; H arom.)

### d) Acide 3-méthoxyfuro[2,3-b]pyrazine-2-carboxylique

Ce composé est obtenu selon le mode opératoire de l'exemple 1 b)
Rdt = 82%, PF = 242°C.
RMN (¹H, DMSO d6) : 4.43 (s; 3H; OCH3), 8.60 (d, J = 2.4Hz ; 1H; H arom.), 8.79 (d, J = 2.4Hz; 1H; H arom.)

### e) 3-Méthoxyfuro[2,3-b]pyrazine-2-carboxylic acid (8-benzyl-8-azabicyclo[3.2.1]oct-3β-yl)amide chlorhydrate

Ce composé est préparé selon le mode opératoire de l'exemple 1 c), mais en utilisant les réactifs correspondants. On l'obtient avec 69% de rendement après chromatographie flash sur silice et chromatographie sur alumine.

### Préparation du sel:

On dissout 1.12 g de la base (2.8 mmol, 1éq) obtenue dans 5 ml d'acétone. On y introduit 0.75 ml d'une solution d'isopropanol - HCl 3.6N (2.8 mmol, 1éq). On évapore sous vide, on reprend dans l'acétone, on filtre le sel formé et on sèche.
Rdt.: 1.02 g (83%).
PF = 222°C
IR : 3359, 1670.9, 1542.7
RMN (base ; ¹H, CDCl₃) : 1.65 (m ; 2H), 1.78 (m ; 2H), 1.95 (m ; 2H), 2.09 (m ; 2H), 3.29 (m ; 2H, H1 et H5) 3.56 (s ; 2H, N CH₂Ph), 4.38 (m ; 1H, H3), 4.55 (s ; 3H; OCH3), 6.70 (m ; 1H; NH), 7.26-7.40 (m ; 5H; Ph), 8.40 (d, J = 2.4Hz ; 1H; H arom.), 8.59 (d, J = 2.4Hz; 1H; H arom.)

| | | |
|---|---|---|
| Analyse | C₂₂ H₂₄ N₄ O₃, HCl, 0.036 H₂O | Masse = 429.570 |

| | %C | %H | %N | %H2O |
|---|---|---|---|---|
| calc. | 61.51 | 5.88 | 13.04 | 0.15 |
| trouv. | 61.26 | 5.88 | 12.70 | 0.15 |

SM (ESI) : 393.2 (M⁺ , 100%)

### Exemple 21:

### 3-Méthoxyfuro[2,3-b]pyrazine-2-carboxylic acid [8-(4-chlorobenzyl)-8-azabicyclo[3.2.1]oct-3β-yl)amide chlorhydrate

Ce composé est préparé selon le mode opératoire de l'exemple 1 c), mais en utilisant les réactifs correspondants, c'est à dire l'acide 3-Méthoxyfuro[2,3-*b*]pyrazine-2-carboxylique décrit dans l'exemple 20 d) et l'amine décrite dans l'exemple 8 d).

On obtient 1.32 g (47%) d'une mousse blanche après chromatographie flash sur silice et chromatographie sur alumine (éluant CH2Cl2).

Le chlorhydrate se prépare dans l'acétone par addition d'isopropanol - HCl.

On obtient 1.27 g (89%) de chlorhydrate.
PF = 231°C
IR : 3385.5, 1677.8, 1541.7
RMN (base ; ¹H, CDCl₃) : 1.63 (m ; 2H), 1.79 (m ; 2H), 1.95 (m ; 2H), 2.07 (m ; 2H), 3.24 (m ; 2H, H1 et H5) 3.51 (s ; 2H, N CH₂Ph), 4.39 (m ; 1H, H3), 4.55 (s ; 3H; OCH3), 6.68 (m ; 1H; NH), 7.26-7.34 (m ; 4H; Ph), 8.40 (d, J = 2.4Hz ; 1H; H arom.), 8.60 (d, J = 2.4Hz; 1H; H arom.)

| | | |
|---|---|---|
| Analyse | C₂₂ H₂₄ N₄ Cl O₃, HCl , 0.08 H₂O | Masse = 464.808 |

| | %C | %H | %N | %H2O |
|---|---|---|---|---|
| calc. | 56.85 | 5.24 | 12.05 | 0.31 |
| trouv. | 56.94 | 5.32 | 12.01 | 0.32 |

SM (ESI) : 427.2 (M⁺ , 100%)

### Exemple 22:

### 3-Méthoxyfuro[2,3-b]pyrazine-2-carboxylic acid [8-(4-fluorobenzyl)-8-azabicyclo[3.2.1]oct-3β-yl)amide chlorhydrate

Ce composé est préparé selon le mode opératoire de l'exemple 1 c), mais en utilisant les réactifs correspondants, c'est à dire l'acide 3-Méthoxyfuro[2,3-*b*]pyrazine-2-carboxylique décrit dans l'exemple 20 d) et l'amine décrite dans l'exemple 11 b).

On obtient 780 mg (46%) d'une mousse blanche après chromatographie flash sur silice et chromatographie sur alumine (éluant CH2Cl2).

Le chlorhydrate se prépare dans l'acétone par addition d'isopropanol - HCl.

On obtient 710 mg (84%) de chlorhydrate.
PF = 234°C
IR : 3401.6, 1670.5, 1521.9
RMN (base ; ¹H, CDCl₃) : 1.63 (m ; 2H), 1.79 (m ; 2H), 1.94 (m ; 2H), 2.07 (m ; 2H), 3.25 (m ; 2H, H1 et H5) 3.51 (s ; 2H, N CH₂Ph), 4.40 (m ; 1H, H3), 4.55 (s ; 3H; OCH3), 6.68 (m ; 1H; NH), 7.01 (m ; 2H; J = 8.4Hz; Ph), 7.34 (m ; 2H; J = 7.64Hz; J' = 5.6Hz; Ph) 8.40 (d, J = 2.4Hz ; 1H; H arom.), 8.59 (d, J = 2.4Hz; 1H; H arom.)

| | | |
|---|---|---|
| Analyse | C₂₂ H₂₃ N₄ F O₃, HCl , 0.19 H₂O | Masse = 450.335 |

| | %C | %H | %N | %H2O |
|---|---|---|---|---|
| calc. | 58.68 | 5.46 | 12.44 | 0.77 |
| trouv. | 58.62 | 5.43 | 12.30 | 0.78 |

SM (ESI) : 411.2 (M⁺ , 100%)

### Exemple 23:

### 3-Méthoxyfuro[2,3-b]pyrazine-2-carboxylic acid [8-thiophèn-2-ylméthyl-8-azabicyclo[3.2.1]oct-3β-yl)amide chlorhydrate

### a) ter-Butyl-N-[8-(2-thiénylméthyl)-8-azabicyclo[3.2.1]oct-3β-yl]carbamate

4.52 g (0.02 mol) de l'intermédiaire *ter*-Butyl-N-(8-azabicyclo[3.2.1]oct-3β-yl)carbamate, obtenu à l'exemple 8 b), sont dissous à 0°C sous azote dans 100 ml de CH2Cl2, avec 2.25 g (0.02 mol) de 2-thiophène carboxaldéhyde et 1.8 ml d'acide acétique. On introduit alors par portions 7 g (1.6 éq) de NaBH(OAc)3 à cette température. Après une nuit de contact à température ambiante sous agitation, on jette le milieu dans l'eau (50 ml), et on extrait 2 fois par du CH2Cl2. Après séchage sur Na2SO4, filtration et évaporation, on triture le résidu avec une solution de NaOH 1N, et on essore les cristaux obtenus, les lave à l'eau puis les sèche sous vide. On obtient 4.85 g (75%) de produit.
PF= 155°C
RMN (¹H, CDCl₃): 1.43 (s; 9H, t-butyl), 1.45 (m; 2H), , 1.69 (m; 2H), 1.82 (m; 2H), 1.98 (m; 2H), 3.26 (m; 2H, H1 et H5), 3.70 (s; 2H, N-CH₂-thioph), 3.79 (m; 1H, H3), 4.32 (m; 1H, NH-carbamate), , 6.87 (m; 1H), 6.98 (m; 1H), 7.20 (m; 1H)

### b) 8-(2-Thiénylméthyl)-8-azabicyclo[3.2.1]oct-3β-amine

Ce composé est obtenu selon le même mode opératoire de l'exemple 8 d).
RMN (¹H, DMSO-d₆) : 1.28-1.34 (m; 4H), 1.47-1.57 (m; 4H), 1.85-1.89 (m; 2H), 2.78 (m; 1H, H3), 3.12 (t, J = 3.0Hz; 2H, H1 et H5), 3.67 (s; 2H, N-CH₂-thioph), 6.94 (m; 2H; H thioph), 7.36 (m; 1H; H thioph).

### c) 3-Méthoxyfuro[2,3-b]pyrazine-2-carboxylic acid [8-thiophèn-2-ylméthyl-8-azabicyclo[3.2.1]oct-3β-yl)amide chlorhydrate

Ce composé est préparé selon le mode opératoire de l'exemple 1 c), mais en utilisant les réactifs correspondants, c'est à dire l'acide 3-Méthoxyfuro[2,3-*b*]pyrazine-2-carboxylique, obtenu à l'exemple 20d), et l'amine précédemment obtenue au stade b) précédent.

On obtient avec un rendement de 52% des cristaux blancs. PF = 143°C.

Le chlorhydrate se prépare dans l'éthanol par addition d'isopropanol - HCl.

On obtient le chlorhydrate avec un rendement de 48%.
PF = 230°C (dec.)
IR : 3360, 1672.8, 1547.7
RMN (base ; ¹H, CDCl₃) : 1.63-1.69 (m ; 2H), 1.79 (m ; 2H), 1.93-1.98 (m ; 2H), 2.05 (m ; 2H), 3.34 (m ; 2H, H1 et H5), 3.73 (s ; 2H; N CH₂-thioph), 4.34-4.45 (m ; 1H; H3), 4.56 (s ; 3H; OCH3), 6.69 (d ; 1H; NH), 6.90-6.96 (m ; 2H; thioph), 7.23 (m ; 1H; thioph) 8.40 (d, J = 2.4Hz ; 1H; H arom.), 8.59 (d, J = 2.4Hz; 1H; H arom.)

| | | |
|---|---|---|
| Analyse | C₂₀ H₂₂ N₄ O₃ S HCl, 0.19 H₂O | Masse = 434.95 |

| | %C | %H | %N |
|---|---|---|---|
| calc. | 55.23 | 5.33 | 12.88 |
| trouv. | 55.33 | 5.36 | 12.70 |

SM (ESI) : 399.26 (M⁺ , 100%)

### Exemple 24:

### 3-Méthylsulfanylfuro[2,3-b]pyrazine-2-carboxylic acid (8-benzyl-8-azabicyclo[3.2.1]oct-3β-yl)amide chlorhydrate

### a) 3-(Diéthoxyphosphoryloxy)furo[2,3-b]pyrazine-2-carboxylic acid éthyl ester

Ce composé est obtenu selon le mode opératoire de l'exemple 2 a), à partir du 3-hydroxy-2-éthoxycarbonylfuro[2,3-*b*]pyrazine, avec un rendement quantitatif, sous forme d'une huile rouge, migrant en CM sur silice avec un Rf=0.46 dans le système acétate d'éthyle-éther de pétrole (80-20).

### b) 3-Méthylsulfanylfuro[2,3-b]pyrazine-2-carboxylic acid éthyl ester

Le produit brut issu du stade a) précédent est traité selon le mode opératoire de l'exemple 2 b), pour fournir un ester qui est engagé dans l'étape suivante sans purification.

### c) Acide 3-Méthylsulfanylfuro[2,3-b]pyrazine-2-carboxylique

Ce dérivé est obtenu selon le mode opératoire de l'exemple 1 b) pour fournir, avec un rendement de 48%, l'acide recherché. Rf=0.11 sur CCM de silice, élution: CH2CL2-Methanol 90-10.
RMN (¹H, DMSO d6) : 2.94 (s; 3H; SCH3), 8.62 (d, J = 2.3Hz ; 1H; H arom.), 8.78 (d, J = 2.3Hz; 1H; H arom.).

### d) 3-Méthylsulfanylfuro[2,3-b]pyrazine-2-carboxylic acid (8-benzyl-8-azabicyclo[3.2.1]oct-3β-yl)amide chlorhydrate

Ce composé est préparé selon le mode opératoire de l'exemple 1 c), mais en utilisant les réactifs correspondants. On l'obtient avec 38% de rendement après chromatographie flash sur silice et chromatographie sur alumine (élution: CH2Cl2), sous forme d'une mousse jaune pâle.

### Préparation du sel:

On dissout 0.63 g de la base (1.5 mmol) obtenue dans 5 ml d'acétone. On y introduit une quantité stoechiométrique d'une solution d'isopropanol - HCl 3.6N. On évapore sous vide, on reprend dans l'acétone, on filtre le sel formé et on sèche. On obtient 520 mg de cristaux jaune pâle.
Rdt. 76%.
PF = 150°C
IR : 3426, 3237, 1649, 1552.
RMN (HCl; ¹H, CD3OD) : 2.11-2.27 (m ; 6H), 2.50 (m ; 2H), 2.94 (s ; 3H; SCH3), 4.00 (m ; 2H, H1 et H5), 4.25 (s ; 2H, N-CH₂Ph), 4.50 (m ; 1H, H3), 7.50 (m ; 3H; Ph), 7.63 (m ; 2H; Ph), 8.47 (d, J = 2.4Hz ; 1H; H arom.), 8.67 (d, J = 2.4Hz; 1H; H arom.)

| | | |
|---|---|---|
| Analyse | C₂₂ H₂₄ N₄ O₂ S, 0.3 HCl , 0.35 H₂O | Masse = 462.22 |

| | %C | %H | %N | %H2O |
|---|---|---|---|---|
| calc. | 57.16 | 5.67 | 12.12 | 1.36 |
| trouv. | 57.35 | 5.74 | 11.51 | 1.47 |

SM (ESI) : 409.2 (M⁺ , 100%)

### Exemple 25:

### 3-Méthylsulfanylfuro[2,3-b]pyrazine-2-carboxylic acid [8-(4-chlorobenzyl)-8-azabicyclo[3.2.1]oct-3β-yl]amide chlorhydrate

Ce composé est préparé selon le mode opératoire de l'exemple 1 c), mais en utilisant les réactifs correspondants. On l'obtient avec 26% de rendement après chromatographie flash sur silice et chromatographie sur alumine (élution: CH2Cl2), sous forme d'une mousse jaune pâle. La CM sur silice donne un Rf=0.26 (élution: CH2Cl2-MeOH 95-5)

### Préparation du sel:

On dissout la base obtenue dans l'acétone. On y introduit une quantité stoechiométrique d'une solution d'isopropanol - HCl 3.6N. On évapore sous vide, on reprend dans l'acétone, triture, et filtre le sel formé puis sèche. On obtient des cristaux jaune pâle. Rdt. 87%.
PF = 173°C
IR : 3398, 3255, 1652, 1560.
RMN (base ; ¹H, CDCl3) : 1.67 (m ; 2H), 1.78 (m ; 2H) 1.93 (m ; 2H), 2.07 (m ; 2H), 2.93 (s ; 3H; SCH3), 3.25 (m ; 2H, H1 et H5), 3.53 (s ; 2H, N-CH₂Ph), 4.40 (m ; 1H, H3), 6.69 (d; 1H; NH), 7.29 (d ; 2H; J = 8.4Hz; Ph), 7.34 (d ; 2H; J = 8.4Hz; Ph), 8.41 (d, J = 2.4Hz ; 1H; H arom.), 8.63 (d, J = 2.4Hz; 1H; H arom.)

| Analyse | | |
|---|---|---|
| | C₂₂ H₂₄ N₄ O₂ S, HCl , 1.14 H₂O | Masse = 499.969 |

| | %C | %H | %N | %H2O |
|---|---|---|---|---|
| calc. | 52.85 | 5.30 | 11.21 | 4.28 |
| trouv. | 52.44 | 5.34 | 11.01 | 4.29 |

SM (ESI) : 443.2 (M⁺ , 100%)

### Exemple 26:

### 3-Méthylsulfanylfuro[2,3-b]pyrazine-2-carboxylic acid [8-(4-fluorobenzyl)-8-azabicyclo[3.2.1]oct-3β-yl]amide chlorhydrate

Ce composé est préparé selon le mode opératoire de l'exemple 1 c), mais en utilisant les réactifs correspondants. On l'obtient avec 48% de rendement après chromatographie flash sur silice et chromatographie sur alumine (élution: CH2Cl2), sous forme d'une mousse jaune pâle. La CCM sur silice donne un Rf=0.32 (élution: CH2Cl2-MeOH 95-5)

### Préparation du sel:

On dissout la base obtenue dans l'acétone. On y introduit une quantité stoechiométrique d'une solution d'isopropanol - HCl 3.6N. On évapore sous vide, on reprend dans l'acétone, triture, et filtre le sel formé puis sèche. On obtient des cristaux jaune pâle. Rdt. 69%.
PF = 182°C
IR : 3421, 3235, 1648, 1553.
RMN (base ; ¹H, CDCl3) : 1.72 (m ; 2H), 1.79 (m ; 2H), 1.92 (m ; 2H), 2.09 (m ; 2H), 2.93 (s ; 3H; SCH3), 3.26 (m ; 2H, H1 et H5), 3.54 (s ; 2H, N-CH₂Ph), 4.40 (m ; 1H, H3), 6.71 (d; 1H; NH), 7.01 (d ; 2H; J = 8.6Hz; Ph), 7.36 (dd ; 2H; J = 8.0Hz et 5.8Hz; Ph), 8.41 (d, J = 2.5Hz ; 1H; H arom.), 8.62 (d, J = 2.5Hz; 1H; H arom.)

| Analyse | | |
|---|---|---|
| | C₂₂ H₂₃ F N₄ O₂ S, 1.1 HCl , 0.75 H₂O | Masse = 480.096 |

| | %C | %H | %N | %H2O |
|---|---|---|---|---|
| calc. | 55.04 | 5.37 | 11.67 | 2.89 |
| trouv. | 54.74 | 5.28 | 11.42 | 2.91 |

SM (ESI) : 427.2 (M⁺ , 100%)

### Exemple 27:

### 3-Méthoxythièno[2,3-b]pyrazine-2-carboxylic acid (8-benzyl-8-azabicyclo[3.2.1]oct-3β-yl)amide chlorhydrate

### a) 3-hydroxy-2-méthoxycarbonylthièno[2,3-b]pyrazine

Le mélange de 1.35 g (7.8 mmol, 1 éq) de 3-chloro-2-pyrazine carboxylate de méthyle préparé à l'exemple 20 a), 0.7 ml (7.8 mmol, 1 éq) de thioglycolate de méthyle et 1.62 g (11.7 mmol, 1.5 éq) de K2CO3 dans 60 ml d'acétonitrile est chauffé sous agitation pendant 16 h. On concentre le solvant et on noie dans 200 ml d'eau. Après avoir acidifier par HCl 1N, on extrait 3 fois par le CH2Cl2, sèche la phase organique sur Na2CO3, filtre et évapore. On obtient 1.21 g (73.5%) de produit cyclisé. PF = 188°C.
RMN (¹H, DMSO) : 2.50 (s ; 3H; SCH3), 3.87 (s ; OCH3),8.81 (d, J = 2.3Hz ; 1H; H arom.), 8.86 (d, J = 2.3Hz; 1H; H arom.) 11.67 (m; 1H; OH)

### b) 3-Méthoxy-2-méthoxycarbonylthièno[2,3-b]pyrazine

Ce composé est obtenu selon le mode opératoire de l'exemple 1 a).
Rdt = 33%. PF = 104°C.
RMN (¹H, CDCl₃) : 3.96 (s ; 3H; OCH3 ester), 4.46 (s; 3H; OCH3), 8.63 (d, J = 2.3Hz ; 1H; H arom.), 8.70 (d, J = 2.3Hz; 1H; H arom.).

### c) Acide 3-méthoxythièno[2,3-b]pyrazine-2-carboxylique

Ce composé est obtenu selon le mode opératoire de l'exemple 1 b).
Rdt = 85%.
RMN (¹H, DMSO d6) : 4.30 (s; 3H; OCH3), 8.80 (d, J = 2.2Hz ; 1H; H arom.), 8.86 (d, J = 2.2Hz; 1H; H arom.).

### d) 3-Méthoxythièno[2,3-b]pyrazine-2-carboxylic acid (8-benzyl-8-azabicyclo[3.2.1]oct-3β-yl)amide chlorhydrate

Ce composé est préparé selon le mode opératoire de l'exemple 1 c), mais en utilisant les réactifs correspondants. On l'obtient avec 68% de rendement après chromatographie flash sur silice (élution CH2Cl2-MeOH 95-5).

### Préparation du sel:

On dissout la base obtenue dans l'acétate d'éthyle. On y introduit stoechiométriquement une solution d'isopropanol - HCl 5N. Après trituration et obtention de cristaux, on filtre le sel formé et on sèche. Rdt. 94%.
PF = 241 °C
IR : 3360, 2957, 2366, 1644, 1536
RMN (base ; ¹H, CDCl₃) : 1.68 (m ; 2H), 1.79 (m ; 2H), 1.96 (m ; 2H), 2.10 (m ; 2H), 3.30 (m ; 2H, H1 et H5) 3.56 (s ; 2H, N CH₂Ph), 4.37 (m ; 1H, H3), 4.50 (s ; 3H; OCH3), 6.70 (m ; 1H; NH), 7.19-7.43 (m ; 5H; Ph), 8.56 (d, J = 2.3Hz ; 1H; H arom.), 8.62 (d, J = 2.3Hz; 1H; H arom.)

| | | |
|---|---|---|
| Analyse | C₂₂ H₂₄ N₄ O₂ S, HCl | Masse = 444.99 |
| | C₂₂ H₂₄ N₄ O₂ S, HCl , 0.11 H₂O | Masse = 446.97 |

| | %C | %H | %N | %H2O |
|---|---|---|---|---|
| calc. | 59.12 | 5.69 | 12.53 | 0.44 |
| trouv. | 59.11 | 5.76 | 12.30 | 0.45 |

SM (ESI) : 409.20 (M⁺ , 100%)

### Exemple 28:

### 3-Méthoxythièno[2,3-b]pyrazine-2-carboxylic acid [8-(4-chorobenzyl)-8-azabicyclo[3.2.1]oct-3β-yl]amide chlorhydrate

Ce composé est préparé selon le mode opératoire de l'exemple 1 c), mais en utilisant les réactifs correspondants. On l'obtient avec 58% de rendement après chromatographie flash sur silice (élution CH2Cl2-MeOH 99-1), sous forme d'une mousse. Rf = 0.31 (CCM silice , éluant: CH2Cl2-MeOH 95-5).

### Préparation du sel:

On dissout la base obtenue dans l'acétate d'éthyle. On y introduit stoechiométriquement une solution d'isopropanol - HCl 5N. Après trituration et obtention de cristaux, on filtre le sel formé et on sèche. Rdt. 87%.
PF = 234°C
IR : 3366, 3032, 2434, 1645, 1534
RMN (base ; ¹H, CDCl₃): 1.67 (m ; 2H), 1.79 (m ; 2H), 1.95 (m ; 2H), 2.07 (m ; 2H), 3.25 (m ; 2H, H1 et H5) 3.51 (s ; 2H, N CH₂Ph), 4.38 (m ; 1H, H3), 4.51 (s ; 3H; OCH3), , 7.28-7.35 (m ; 4H; Ph), 7.40 (m ; 1H; NH), 8.56 (d, J = 2.3Hz ; 1H; H arom.), 8.63 (d, J = 2.3Hz; 1H; H arom.)

| | | |
|---|---|---|
| Analyse | C₂₂ H₂₃ Cl N₄ O₂ S, HCl | Masse = 479.43 |
| | C₂₂ H₂₃ Cl N₄ O₂ S, HCl , 0.15 H₂O | Masse = 482.13 |

| | %C | %H | %N | %H2O |
|---|---|---|---|---|
| calc. | 54.81 | 5.08 | 11.62 | 0.56 |
| trouv. | 54.64 | 5.33 | 11.30 | 0.55 |

SM (ESI) : 443.1 (M⁺ , 100%)

### Exemple 29:

### 3-Méthoxythièno[2,3-b]pyrazine-2-carboxylic acid [8-(4-fluorobenzyl)-8-azabicyclo[3.2.1]oct-3β-yl]amide chlorhydrate

Ce composé est préparé selon le mode opératoire de l'exemple 1 c), mais en utilisant les réactifs correspondants. On l'obtient avec 80% de rendement après chromatographie flash sur silice (élution CH2Cl2-MeOH 99-1), sous forme d'une mousse. Rf = 0.17 (CCM silice , éluant: CH2Cl2-MeOH 95-5).

### Préparation du sel:

On dissout la base obtenue dans l'acétate d'éthyle. On y introduit stoechiométriquement une solution d'isopropanol - HCl 5N. Après trituration et obtention de cristaux, on filtre le sel formé et on sèche. Rdt. 87%.
PF = 233°C
IR : 3369, 2499, 2465, 2432, 1644, 1533
RMN (base ; ¹H, CDCl₃) : 1.64 (m ; 2H), 1.79 (m ; 2H), 1.96 (m ; 2H), 2.08 (m ; 2H), 3.26 (m ; 2H, H1 et H5) 3.51 (s ; 2H, N CH₂Ph), 4.38 (m ; 1H, H3), 4.50 (s ; 3H; OCH3), 7.01 (t ; J = 8.5Hz; 2H; Ph), 7.33-7.41 (m ; 3H; NH et Ph), 8.56 (d, J = 2.3Hz ; 1H; H arom.), 8.625 (d, J = 2.3Hz; 1H; H arom.)

| | | |
|---|---|---|
| Analyse | C₂₂ H₂₃ F N₄ O₂ S, HCl | Masse = 462.98 |
| | C₂₂ H₂₃ F N₄ O₂ S, HCl , 0.12 H₂O | Masse = 465.14 |

| | %C | %H | %N | %H2O |
|---|---|---|---|---|
| calc. | 56.81 | 5.25 | 12.04 | 0.47 |
| trouv. | 56.58 | 5.36 | 11.67 | 0.48 |

SM (ESI) : 427.19 (M⁺ , 100%)

### Exemple 30:

### 3-Méthylsulfanylthièno[2,3-b]pyrazine-2-carboxylic acid (8-benzyl-8-azabicyclo[3.2.1]oct-3β-yl)amide chlorhydrate

### a) 3-(Diéthoxyphosphoryloxy)thièno[2,3-b]pyrazine-2-carboxylic acid méthyl ester

Ce composé est obtenu selon le mode opératoire de l'exemple 2 a), à partir du produit de l'exemple 27 a), avec un rendement de 46%, sous forme d'une huile, migrant en CCM sur silice avec un Rf=0.33 dans le système acétate d'éthyle-éther de pétrole (50-50).

### b) 3-Méthylsulfanylthièno[2,3-b]pyrazine-2-carboxylic acid méthyl ester

Le produit brut issu du stade a) précédent est traité selon le mode opératoire de l'exemple 2 b), pour fournir un ester qui est engagé dans l'étape suivante sans purification.

### c) Acide 3-Méthylsulfanylthièno[2,3-b]pyrazine-2-carboxylique

Ce dérivé est obtenu selon le mode opératoire de l'exemple 1 b) pour fournir avec un rendement de 90% l'acide recherché. Rf=0.19 sur CCM de silice, élution: CH2CL2-Methanol 90-10.
RMN (¹H, DMSO d6) : 2.88 (s; 3H; SCH3), 8.81 (d, J = 2.3Hz ; 1H; H arom.), 8.87 (d, J = 2.3Hz; 1H; H arom.).

### d) 3-Méthylsulfanylthièno[2,3-b]pyrazine-2-carboxylic acid (8-benzyl-8-azabicyclo[3.2.1]oct-3β-yl)amide chlorhydrate

Ce composé est préparé selon le mode opératoire de l'exemple 1 c), mais en utilisant les réactifs correspondants. On l'obtient avec 49% de rendement après chromatographie flash sur silice et chromatographie sur alumine (élution: CH2Cl2), sous forme de cristaux jaune pâle.

### Préparation du sel:

On dissout la base obtenue dans l'acétate d'éthyle, on y introduit une quantité stoechiométrique d'une solution d'isopropanol - HCl 5N. On évapore sous vide, on reprend dans l'acétate d'éthyle, on filtre le sel formé et on sèche. On obtient des cristaux jaune pâle. Rdt. 89%.
PF = 249°C
RMN (base ; ¹H, CDCl3) : 1.80 (m ; 4H), 1.99 (m ; 2H), 2.11 (m ; 2H), 2.64 (s ; 3H; SCH3), 3.31 (m ; 2H, H1 et H5), 3.57 (s ; 2H, N-CH₂Ph), 4.42 (m ; 1H, H3), 7.24-7.46 (m ; 5H; Ph), 8.62 (d, J = 2.3Hz ; 1H; H arom.), 8.64 (m ; 1H; NH), 8.75 (d, J = 2.3Hz; 1H; H arom.)

| | | |
|---|---|---|
| Analyse | C₂₂ H₂₄ N₄ O S₂, HCl | Masse = 461.05 |
| | C₂₂ H₂₄ N₄ O S₂, HCl , 0.074 H₂O | Masse = 462.38 |

| | %C | %H | %N | %H2O |
|---|---|---|---|---|
| calc. | 57.15 | 5.48 | 12.12 | 0.29 |
| trouv. | 57.05 | 5.37 | 11.96 | 0.29 |

SM (ESI) : 425.1 (M⁺ , 100%)

### Exemple 31:

### 3-Méthylsulfanylthièno[2,3-b]pyrazine-2-carboxylic acid [8-(4-chorobenzyl)-8-azabicyclo[3.2.1]oct-3β-yl]amide chlorhydrate

Ce composé est préparé selon le mode opératoire de l'exemple 1 c), mais en utilisant les réactifs correspondants. On l'obtient avec 38% de rendement après chromatographie flash sur silice (élution CH2Cl2-MeOH 99-1), sous forme d'une mousse. Rf = 0.39 (CCM silice , éluant: CH2Cl2-MeOH 95-5).

### Préparation du sel:

On dissout la base obtenue dans l'acétate d'éthyle. On y introduit stoechiométriquement une solution d'isopropanol - HCl 5N. Après trituration et obtention des cristaux blancs, on filtre le sel formé et on sèche. Rdt. 68%.
PF = 236°C
IR : 3466, 3254, 2515, 1654, 1637, 1545
RMN (base ; ¹H, CDCl₃) : 1.65-1.81 (m ; 4H), 2.00 (m ; 2H), 2.09 (m ; 2H), 2.65 (s ; 3H, SCH3), 3.27 (m ; 2H, H1 et H5) 3.53 (s ; 2H, N CH₂Ph), 4.41 (m ; 1H, H3), 7.30 (d ; J = 8.3Hz, 2H; Ph), 7.34 (d ; J = 8.3Hz, 2H; Ph), 8.2 (d, J = 2.3Hz ; 1H; H arom.), 8.63 (m ; 1H; NH), 8.76 (d, J = 2.3Hz; 1H; H arom.)

| | | |
|---|---|---|
| Analyse | C₂₂ H₂₃ Cl N₄ O S₂, HCl | Masse = 495.50 |
| | C₂₂ H₂₃ Cl N₄ O S₂, HCl , 0.38 H₂O | Masse = 502.34 |

| | %C | %H | %N | %H2O |
|---|---|---|---|---|
| calc. | 52.60 | 4.97 | 11.15 | 1.38 |
| trouv. | 52.44 | 5.16 | 10.98 | 1.39 |

SM (ESI) : 459.1 (M⁺ , 100%)

### Exemple 32:

### 3-Méthylsulfanylthièno[2,3-b]pyrazine-2-carboxylic acid [8-(4-fluorobenzyl)-8-azabicyclo[3.2.1]oct-3β-yl]amide chlorhydrate

Ce composé est préparé selon le mode opératoire de l'exemple 1 c), mais en utilisant les réactifs correspondants. On l'obtient avec 38% de rendement après chromatographie flash sur silice (élution CH2Cl2-MeOH 99-1), sous forme d'une mousse. Rf = 0.20 (CCM silice , éluant: CH2Cl2-MeOH 95-5).

### Préparation du sel:

On dissout la base obtenue dans l'acétate d'éthyle. On y introduit stoechiométriquement une solution d'isopropanol - HCl 5N. Après trituration et obtention des cristaux blancs, on filtre le sel formé et on sèche. Rdt. 86%.
PF = 221°C
IR : 3426, 32393, 3169, 3051, 2972, 1636, 1608, 1560
RMN (base ; ¹H, CDCl₃) : 1.57-1.81 (m ; 4H), 1.99 (m ; 2H), 2.09 (m ; 2H), 2.64 (s ; 3H; SCH3), 3.28 (m ; 2H, H1 et H5) 3.52 (s ; 2H, N CH₂Ph), 4.40 (m ; 1H, H3), , 7.01 (d ; J = 8.5Hz, 2H; Ph), 7.36 (dd ; J = 5.9Hz, 2H; Ph), 8.62 (d, J = 2.3Hz ; 1H; H arom.), 8.62 (m ; 1H; NH), 8.75 (d, J = 2.3Hz; 1H; H arom.)

| | | |
|---|---|---|
| Analyse | C₂₂ H₂₃ F N₄ O S₂, HCl | Masse = 479.04 |
| | C₂₂ H₂₃ F N₄ O S₂, HCl , 0.35 H₂O | Masse = 485.35 |

| | %C | %H | %N | %H2O |
|---|---|---|---|---|
| calc. | 54.44 | 5.13 | 11.54 | 1.32 |
| trouv. | 54.23 | 5.40 | 11.40 | 1.31 |

SM (ESI) : 443.16 (M⁺ , 100%)

### Exemple 33:

### 3-Méthylsulfanylthièno[2,3-b]pyrazine-2-carboxylic acid [8-thiophèn-2-ylméthyl-8-azabicyclo[3.2.1]oct-3β-yl)amide chlorhydrate

### a) ter-Butyl-N-[8-(thiophèn-2-ylméthyl)-8-azabicyclo[3.2.1]oct-3β-yl]carbamate

4.52 g (0.02 mol) de l'intermédiaire *ter*-Butyl-N-(8-azabicyclo[3.2.1]oct-3β-yl)carbamate obtenu à l'exemple 8 b) sont dissous à 0°C sous azote dans 100 ml de CH2Cl2, avec 2.25 g (0.02 mol) de 2-thiophène carboxaldéhyde et 1.8 ml d'acide acétique. On introduit alors par portions 7 g (1.6 éq) de NaBH(OAc)3 à cette température. Après une nuit de contact à température ambiante sous agitation, on jette le milieu dans l'eau (50 ml), et on extrait 2 fois par du CH2Cl2. Après séchage sur Na2SO4, filtration et évaporation, on triture le résidu avec une solution de NaOH 1N, et on essore les cristaux obtenus, les lave à l'eau puis les sèche sous vide. On obtient 4.85 g (75%) de produit.
PF= 155°C
RMN (¹H, CDCl₃): 1.43 (s; 9H, t-butyl), 1.45 (m; 2H), , 1.69 (m; 2H), 1.82 (m; 2H), 1.98 (m; 2H), 3.26 (m; 2H, H1 et H5), 3.70 (s; 2H, N-CH₂-thioph), 3.79 (m; 1H, H3), 4.32 (m; 1H, NH-carbamate), , 6.87 (m; 1H), 6.98 (m; 1H), 7.20 (m; 1H)

### b) 8-(thiophèn-2-ylméthyl)-8-azabicyclo[3.2.1]oct-3β-amine

Ce composé est obtenu selon le même mode opératoire de l'exemple 8 d).
RMN (¹H, DMSO-d₆) : 1.28-1.34 (m; 4H), 1.47-1.57 (m; 4H), 1.85-1.89 (m; 2H), 2.78 (m; 1H, H3), 3.12 (t, J = 3.0Hz; 2H, H1 et H5), 3.67 (s; 2H, N-CH₂-thioph), 6.94 (m; 2H; H thioph), 7.36 (m; 1H; H thioph).

### c) 3-Méthylsulfanylthièno[2,3-b]pyrazine-2-carboxylic acid [8-thiophèn-2-ylméthyl-8-azabicyclo[3.2.1]oct-3β-yl)amide chlorhydrate

Ce composé est préparé selon le mode opératoire de l'exemple 1 c), mais en utilisant les réactifs correspondants, c'est à dire l'acide 3-Méthylsulfanylthièno[2,3-*b*]pyrazine-2-carboxylique et l'amine obtenue à l'exemple 23 b) .

On obtient avec un rendement de 29% une mousse.

Le chlorhydrate se prépare dans l'acétate d'éthyle par addition d'isopropanol - HCl.

On obtient le chlorhydrate avec un rendement de 97%.
PF = 250°C
IR : 3425, 3235, 1642.9, 1537
RMN (base ; ¹H, CDCl₃) : 1.72-1.81 (m ; 4H), 1.99-2.08 (m ; 4H), 2.64 (s ; 3H, SCH3), 3.37 (m ; 2H, H1 et H5), 3.75 (s ; 2H; N CH₂-thioph), 4.35-4.45 (m ; 1H; H3), 6.92-6.96 (m ; 2H; thioph), 7.24 (m ; 1H; thioph), 8.63 (d, J = 2.3Hz ; 1H; H arom.), 8.64 (d ; 1H; NH), 8.76 (d, J = 2.3Hz; 1H; H arom.)

| | | |
|---|---|---|
| Analyse | C₂₀ H₂₂ N₄ O S₃ , HCl | Masse = 467.08 |
| | C₂₀ H₂₂ N₄ O S₃ , 1.15 HCl , 0.26 H₂O | Masse = 477.23 |

| | %C | %H | %N | H2O |
|---|---|---|---|---|
| calc. | 50.34 | 5.00 | 11.74 | 0.98 |
| trouv. | 50.33 | 5.00 | 11.61 | 0.96 |

SM (ESI) : 431.2 (M⁺ , 100%)

## Revendications

1. Les composés de formule générale 1 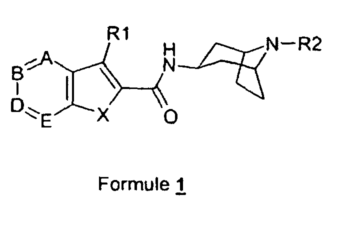 dans laquelle :
A, B, D et E représentent un ou deux atomes d'azote , les autres étant des atomes de carbone,
X représente un S, ou un O, formant ainsi un système hétéroaromatique fusionné bicyclique, tel que le thièno[2,3-b]pyridine, le furo[2,3-b]pyridine, le thièno[3,2-b]pyridine, le furo[3,2-b]pyridine, le thièno[2,3-b]pyrazine, le furo[2,3-b]pyrazine, le thièno[2,3-c]pyridine, le furo[2,3-c]pyridine, le thièno[3,2-c]pyridine et le furo[3,2-c]pyridine,
R1 représente un groupe alcoxy en C1-C6, linéaire ou ramifié, un groupe alkyl thio en C1-C6, liméaire ou ramifié,
R2 représente un groupe alkyl linéaire, ramifié, cyclique en C2-C8, un groupe 2- ou 3- thiènylméthyl, soit un groupe benzyl éventuellement substitué par un ou plusieurs substituants comme halogènes, F, Cl, Br, I, alkyl en C1-4, alcoxy en C1-C4, CF3, CN, NO2, OH,
et leurs sels pharmaceutiquement acceptables.

2. Les composés de formule générale 1, selon la revendication 1, **caractérisés en ce que** le groupement A, B, D, E, X représentent les motifs hétérocycliques suivants: furo[3,2-b]pyridine, thièno[3,2-c]pyridine, thièno[2,3-c]pyridine, thièno[3,2-b]pyridine, thièno[2,3-b]pyridine, thièno[2,3-b]pyrazine, et le groupement R1 représente un méthoxy ou un éthoxy, le groupement R2 représente un cyclohexylméthyl, un thiophène-2-ylméthyl, un thiophène-3-ylméthyl, un benzyl non substitué ou un benzyl substitué par un ou plusieurs F, Cl, OMe, CN.

3. Les composés de formule **1****,** selon les revendications 1 et 2 **caractérisés en ce qu'**ils sont choisis parmi le groupe suivant:
3-Méthoxythièno[2,3-*b*]pyridine-2-carboxylic acid (8-benzyl-8-azabicyclo[3.2.1]oct-3β-yl)amide
3-Méthoxythièno[3,2-*b*]pyridine-2-carboxylic acid (8-benzyl-8-azabicyclo[3.2.1]oct-3β-yl)amide
3-Méthoxyfuro[3,2-*b*]pyridine-2-carboxylic acid (8-benzyl-8-azabicyclo[3.2.1]oct-3β-yl)amide
3-Méthoxythièno[3,2-*c*]pyridine-2-carboxylic acid (8-benzyl-8-azabicylo[3.2.1]oct-3β-yl)amide
3-Méthoxythièno[2,3-*b*]pyridine-2-carboxylic acid [8-(4-chlorobenzyl)-8-azabicyclo[3.2.1]oct-3β-yl] amide
3-Méthoxythièno[2,3-*b*]pyridine-2-carboxylic acid [8-(4-méthoxybenzyl)-8-azabicyclo[3.2.1]oct-3β-yl] amide
3-Méthoxythièno[2,3-*b*]pyridine-2-carboxylic acid [8-cyclohexylméthyl-8-azabicyclo[3.2.1]oct-3β-yl] amide
3-Méthoxythièno[2,3-*b*]pyridine-2-carboxylic acid [8-(4-fluorobenzyl)-8-azabicyclo[3.2.1]oct-3β-yl] amide
3-Méthoxythièno[2,3-*b*]pyridine-2-carboxylic acid [8-thiophèn-3-ylméthyl-8-azabicyclo[3.2.1]oct-3β-yl] amide
3-Méthoxythièno[2,3-*c*]pyridine-2-carboxylic acid (8-benzyl-8-azabicyclo[3.2.1]oct-3β-yl]amide
3-Isopropoxythièno[2,3-*b*]pyridine-2-carboxylic acid (8-benzyl-8-azabicyclo[3.2.1]oct-3β-yl]amide
3-Éthoxythièno[2,3-*b*]pyridine-2-carboxylic acid (8-benzyl-8-azabicyclo[3.2.1]oct-3β-yl)amide
3-Méthoxyfuro[3,2-*b*]pyridine-2-carboxylic acid [8-(4-fluorobenzyl)-8-azabicyclo[3.2.1]oct-3β-yl]amide
3-Méthoxyfuro[3,2-*b*]pyridine-2-carboxylic acid [8-(4-chlorobenzyl)-8-azabicyclo[3.2.1]oct-3β-yl]amide
3-Méthoxyfuro[3,2-*b*]pyridine-2-carboxylic acid (8-cyclohexylméthyl-8-azabicyclo[3.2.1]oct-3β-yl)amide
3-Méthylsulfanylthièno[2,3-*b*]pyridine-2-carboxylic acid (8-benzyl-8-azabicyclo[3.2.1]oct-3β-yl)amide
3-Méthoxythièno[2,3-*b*]pyrazine-2-carboxylic acid (8-benzyl-8-azabicyclo[3.2.1]oct-3β-yl)amide
3-Méthoxythièno[2,3-*b*]pyrazine-2-carboxylic acid [8-(4-chlorobenzyl)-8-azabicyclo[3.2.1]oct-3β-yl]amide
3-Méthylsulfanylthièno[2,3-*b*]pyrazine-2-carboxylic acid (8-benzyl-8-azabicyclo[3.2.1]oct-3β-yl)amide
3-Méthylsulfanylthièno[2,3-*b*]pyrazine-2-carboxylic acid [8-(4-chlorobenzyl)-8-azabicyclo[3.2.1]oct-3β-yl]amide
3-Méthoxythièno[2,3-*b*]pyrazine-2-carboxylic acid [8-(4-fluorobenzyl)-8-azabicyclo[3.2.1]oct-3β-yl]amide
3-Méthylsulfanylthièno[2,3-*b*]pyrazine-2-carboxylic acid [8-(4-fluorobenzyl)-8-azabicyclo[3.2.1]oct-3β-yl]amide
3-Méthoxythièno[2,3-*b*]pyrazine-2-carboxylic acid (8-thiophèn-2-ylméthyl-8-azabicyclo[3.2.1]oct-3β-yl)amide
3-Méthoxyfuro[2,3-*b*]pyrazine-2-carboxylic acid (8-benzyl-8-azabicyclo[3.2.1]oct-3β-yl)amide
3-Méthoxyfuro[2,3-*b*]pyrazine-2-carboxylic acid [8-(4-chlorobenzyl)-8-azabicyclo[3.2.1]oct-3β-yl]amide
3-Méthoxyfuro[2,3-*b*]pyrazine-2-carboxylic acid [8-(4-fluorobenzyl)-8-azabicyclo[3.2.1]oct-3β-yl]amide

4. Procédé de préparation des composés de formule générale **1**, selon l'une des revendications 1 à 3, **caractérisé en ce que** l'on fait réagir un acide hétérocyclique de formule **2**, 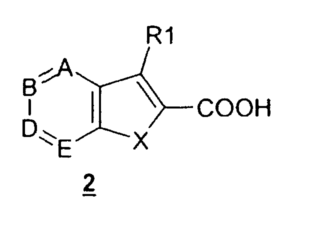 avec une amine polycyclique de formule **3**, 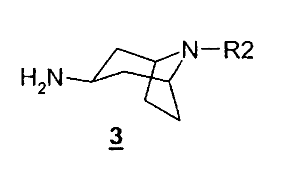 formules dans lesquelles les différents radicaux A, B, D, E, X, R1 et R2 ont les significations données dans la revendication 1, dans les conditions de couplage avec l'action du chloroformiate d'alkyle en présence de triéthylamine, dans le chlorure de méthylène, à basse température ou bien par action du chlorure d'acide de l'acide hétérocyclique correspondant.

5. Les composés de formule **4**, comme intermédiaires de synthèse, dans laquelle X est un O ou S, R1 un OH, un alcoxy en C1-6, ou un thioalcoxy en C1-6, et R représente un hydrogène, sauf dans le cas où R1 est OH, un méthyl ou un éthyl.

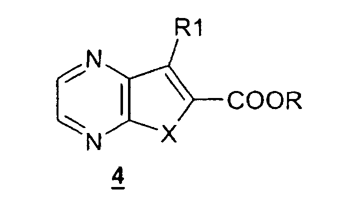

6. Les composés de formule 4, selon la revendication 5, **caractérisés en ce qu'**ils sont choisis parmi :
3-Hydroxy-2méthoxycarbonylfuro[2,3-b]pyrazine
3-méthylsulfanyl-2méthoxycarbonylfuro[2,3-b]pyrazine
acide 3-méthoxy-furo[2,3-b]pyrazine-2-carboxylique
acide 3-éthoxy-furo[2,3-b]pyrazine-2-carboxylique
acide 3-méthylsulfanylfuro[2,3-b]pyrazine-2-carboxylique
3-Hydroxy-2méthoxycarbonylthièno[2,3-b]pyrazine
3-méthylsulfanyl-2méthoxycarbonylthièno[2,3-b]pyrazine
acide 3-méthoxythièno[2,3-b]pyrazine-2-carboxylique
acide 3-éthoxythièno[2,3-b]pyrazine-2-carboxylique
acide 3-méthylsulfanylthièno[2,3-b]pyrazine-2-carboxylique.

7. A titre de médicament les composé de formule générale **I** selon l'une des revendications 1 à 3.

8. Composition pharmaceutique, **caractérisée en ce qu'**elle comprend au moins un composé de formule 1 selon l'une des revendications 1 à 3, et un excipient approprié, notamment pour une administration orale ou parentérale, en particulier à une dose journalière de 0,1 à 100 mg.

9. Utilisation d'un composé de formule 1 selon l'une des revendications 1 à 3, pour la préparation d'un médicament destiné au traitement de la schizophrénie, de ses symptômes positifs ou négatifs ou des troubles du système nerveux central sensible au traitement antidopaminergique comme les désordres obsessionnels compulsifs, l'anxiété, la dépression, la toxicomanie, des dyskinésies tardives, de l'autisme, des désordres gastrointestinaux.

## Patentansprüche

1. Verbindungen der allgemeinen Formel 1 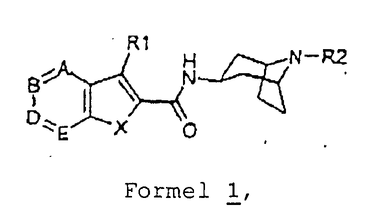 in welcher:
A, B, D und E für ein oder zwei Stickstoffatome stehen, wobei die anderen Kohlenstoffatome sind,
X für ein S oder ein O steht, wodurch ein kondensiertes bicyclisches heteroaromatisches System, wie Thieno[2,3-b]pyridin, Furo[2,3-b]pyridin, Thieno[3,2-b]pyridin, Furo[3,2-b]pyridin, Thieno[2,3-b]pyrazin, Furo[2,3-b]pyrazin, Thieno[2,3-c]pyridin, Furo[2,3-c]pyridin, Thieno[3,2-c]pyridin und Furo[3,2-c]-pyridin, gebildet wird,
R1 für eine lineare oder verzweigte C₁-C₆-Alkoxygruppe, eine lineare oder verzweigte C₁-C₆-Alkylthiogruppe steht,
R2 für eine lineare, verzweigte, cyclische C₂-C₈-Alkylgruppe, eine 2- oder 3-Thienylmethylgruppe oder eine Benzylgruppe, die gegebenenfalls durch einen oder mehrere Substituenten, wie Halogene, F, Cl, Br, I, C₁₋₄-Alkyl, C₁₋₄-Alkoxy, CF₃, CN, NO₂, OH, substituiert ist, steht,
und deren pharmazeutisch verträgliche Salze.

2. Verbindungen der allgemeinen Formel 1 nach Anspruch 1, **dadurch gekennzeichnet, dass** die Gruppen A, B, D, E, X für die folgenden heterocyclischen Motive stehen: Furo[3,2-b]pyridin, Thieno[3,2-c]pyridin, Thieno[2,3-b]pyridin, Thieno[3,2-b]pyridin, Thieno[2,3-b]pyridin, Thieno[2,3-b]pyrazin, und die Gruppe R1 für Methoxy oder Ethoxy steht, die Gruppe R2 für Cyclohexylmethyl, Thiophen-2-ylmethyl, Thiophen-3-ylmethyl, nicht-substituiertes Benzyl oder ein durch.ein oder mehrere F, Cl, OMe, CN substituiertes Benzyl steht.

3. Verbindungen der Formel **1** nach den Ansprüchen 1 und 2, **dadurch gekennzeichnet, dass** sie ausgewählt werden aus der folgenden Gruppe:
3-Methoxythieno[2,3-*b*]pyridin-2-carbonsäure-(8-benzyl-8-azabicyclo[3.2.1]oct-3β-yl)amid
3-Methoxythieno[3,2-*b*]pyridin-2-carbonsäure-(8-benzyl-8-azabicyclo[3.2.1]oct-3β-yl)amid
3-Methoxyfuro[3,2-*b*]pyridin-2-carbonsäure-(8-benzyl-8-azabicyclo[3.2.1]oct-3β-yl)amid
3-Methoxythieno[3,2-*c*]pyridin-2-carbonsäure-(8-benzyl-8-azabicyclo[3.2.1]oct-3β-yl)amid
3-Methoxythieno[2,3-*b*]pyridin-2-carbonsäure-[8-(4-chlorbenzyl)-8-azabicyclo[3.2.1]oct-3β-yl]amid
3-Methoxythieno[2,3-*b*]pyridin-2-carbonsäure-[8-(4-methoxybenzyl)-8-azabicyclo[3.2.1]oct-3β-yl]amid
3-Methoxythieno[2,3-*b*]pyridin-2-carbonsäure-[8-cyclohexylmethyl-8-azabicyclo[3.2.1]oct-3β-yl]amid
3-Methoxythieno[2,3-*b*]pyridin-2-carbonsäure-[8-(4-fluorbenzyl)-8-azabicyclo[3.2.1]oct-3β-yl]amid
3-Methoxythieno[2,3-*b*]pyridin-2-carbonsäure-[8-thiophen-3-ylmethyl-8-azabicyclo[3.2.1]oct-3β-yl]amid
3-Methoxythieno[2,3-*c*]pyridin-2-carbonsäure-(8-benzyl-8-azabicyclo[3.2.1]oct-3β-yl)amid
3-Isopropoxythieno[2,3-*b*]pyridin-2-carbonsäure-(8-benzyl-8-azabicyclo[3.2.1]oct-3β-yl)amid
3-Ethoxythieno[2,3-*b*]pyridin-2-carbonsäure-(8-benzyl-8-azabicyclo[3.2.1]oct-3β-yl)amid
3-Methoxyfuro[3,2-*b*]pyridin-2-carbonsäure-[8-(4-fluorbenzyl)-8-azabicyclo[3.2.1]oct-3β-yl]amid
3-Methoxyfuro[3,2-*b*]pyridin-2-carbonsäure-[8-(4-chlorbenzyl)-8-azabicyclo[3.2.1]oct-3β-yl]amid
3-Methoxyfuro[3,2-*b*]pyridin-2-carbonsäure-(8-cyclohexylmethyl-8-azabicyclo[3.2.1]oct-3β-yl)amid
3-Methylsulfanylthieno[2,3-*b*]pyridin-2-carbonsäure-(8-benzyl-8-azabicyclo[3.2.1]oct-3β-yl)amid
3-Methoxythieno[2,3-*b*]pyrazin-2-carbonsäure-(8-benzyl-8-azabicyclo[3.2.1]oct-3β-yl)amid
3-Methoxythieno[2,3-*b*]pyrazin-2-carbonsäure-[8-(4-chlorbenzyl)-8-azabicyclo[3.2.1]oct-3β-yl]amid
3-Methylsulfanylthieno[2,3-*b*]pyrazin-2-carbonsäure-(8-benzyl-8-azabicyclo[3.2.1]oct-3β-yl)amid
3-Methylsulfanylthieno[2,3-*b*]pyrazin-2-carbonsäure-[8-(4-chlorbenzyl)-8-azabicyclo[3.2.1]oct-3β-yl]amid
3-Methoxythieno[2,3-*b*]pyrazin-2-carbonsäure-[8-(4-fluorbenzyl)-8-azabicyclo[3.2.1]oct-3β-yl]amid
3-Methylsulfanylthieno[2,3-*b*]pyrazin-2-carbonsäure-[8-(4-fluorbenzyl)-8-azabicyclo[3.2.1]oct-3β-yl]amid
3-Methoxythieno[2,3-*b*]pyrazin-2-carbonsäure-(8-thiophen-2-ylmethyl-8-azabicyclo[3.2.1]oct-3β-yl)amid
3-Methoxyfuro[2,3-*b*]pyrazin-2-carbonsäure-(8-benzyl-8-azabicyclo[3.2.1]oct-3β-yl)amid
3-Methoxyfuro[2,3-*b*]pyrazin-2-carbonsäure-[8-(4-chlorbenzyl)-8-azabicyclo[3.2.1]oct-3β-yl]amid
3-Methoxyfuro[2,3-*b*]pyrazin-2-carbonsäure-[8-(4-fluorbenzyl)-8-azabicyclo[3.2.1]oct-3β-yl]amid

4. Verfahren zur Herstellung der Verbindungen der allgemeinen Formel **1** nach einem der Ansprüche 1 bis 3, **dadurch gekennzeichnet, dass** man eine heterocyclische Säure der Formel **2** 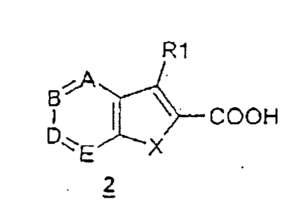 mit einem polycyclischen Amin der Formel **3** 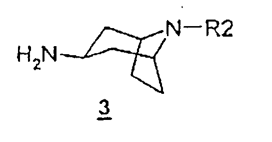 in welchen Formeln die verschiedenen Reste A, B, D, E, X, R1 und R2 die in Anspruch 1 angegebenen Bedeutungen haben, reagieren lässt unter Kopplungsbedingungen unter Einwirkung des Alkylchlorformiats in Gegenwart von Triethylamin in Methylenchlorid bei niedriger Temperatur oder ebenso unter Einwirkung des Säurechlorids der entsprechenden heterocyclischen Säure.

5. Verbindungen der Formel **4** als Synthese-Zwischenprodukte, in welcher X ein O oder S ist, R1 ein OH, ein C₁₋₆-Alkoxy oder ein C₁₋₆-Thioalkoxy ist und R für ein Wasserstoffatom steht, bis auf den Fall, wo R1 OH, Methyl oder Ethyl ist.

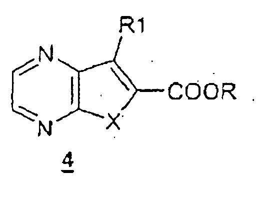

6. Verbindungen der Formel 4 nach Anspruch 5, **dadurch gekennzeichnet, dass** sie ausgewählt werden aus:
3-Hydroxy-2-methoxycarbonylfuro[2,3-b]pyrazin
3-Methylsulfanyl-3-methoxycarbonylfuro[2,3-b]pyrazin
3-Methoxyfuro[2,3-b]pyrazin-2-carbonsäure
3-Ethoxyfuro[2,3-b]pyrazin-2-carbonsäure
3-Methylsulfanylfuro[2,3-b]pyrazin-2-carbonsäure
3-Hydroxy-2-methoxycarbonylthieno[2,3-b]pyrazin
3-Methylsulfanyl-2-methoxycarbonylthieno[2,3-b]pyrazin
3-Methoxythieno[2,3-b]pyrazin-2-carbonsäure
3-Ethoxythieno[2,3-b]pyrazin-2-carbonsäure
3-Methylsulfanylthieno[2,3-b]pyrazin-2-carbonsäure.

7. Verbindungen der allgemeinen Formel **I** nach einem der Ansprüche 1 bis 3 als Arzneimittel.

8. Pharmazeutische Zusammensetzung, **dadurch gekennzeichnet, dass** sie wenigstens eine Verbindung der Formel **1** nach einem der Ansprüche 1 bis 3 und einen geeigneten Träger, insbesondere für eine orale oder parenterale Verabreichung, insbesondere in einer täglichen Dosis von 0,1 bis 100 mg enthält.

9. Verwendung einer Verbindung der Formel **1** nach einem der Ansprüche 1 bis 3 für die Herstellung eines Arzneimittels, welches für die Behandlung von Schizophrenie, deren positiven oder negativen Symptomen oder von Störungen des Zentralnervensystems, das gegenüber einer antidopaminergen Behandlung empfindlich ist, wie obsessiv-kompulsiven Erkrankungen, Angst, Depressionen, Drogenabhängigkeit, dystonen Syndromen, Autismus, gastrointestinalen Erkrankungen bestimmt ist.

## Claims

1. Compounds of general formula 1 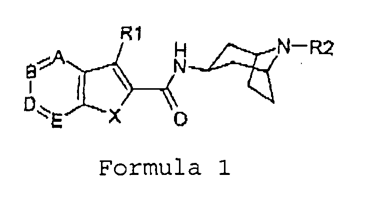 wherein:
A, B, D and E represent one or two nitrogen atoms, the others being carbon atoms,
X represents an S, or an O, thus forming a bicyclic merged heteroaromatic system such as thieno[2,3-b]pyridine, furo[2,3-b]pyridine, thieno[3,2-b]pyridine, furo[3,2-b]pyridine, thieno[2,3-b]pyrazine, furo[2,3-b]pyrazine, thieno[2,3-c]pyridine, furo[2,3-c]pyridine, thieno[3,2-c]pyridine and furo[3,2-c]pyridine,
R1 represents a linear or branched C1-C6 alcoxy group, a linear or branched C1-C6 thio alkyl group,
R2 represents a C2-C8 linear, branched, cyclic alkyl group, a 2- or 3-thienylmethyl group, i.e. a benzyl group possibly substituted by one or more substituents such as halogens, F, Cl, Br, I, C1-4 alkyl, C1-C4 alcoxy, CF3, CN, NO2, OH,
and their pharmaceutically acceptable salts.

2. The compounds of general formula 1, according to claim 1, **characterized in that** the A, B, D, E, X groups represent the following heterocyclic systems: furo[3,2-b]pyridine, thieno[3,2-c]pyridine, thieno[2,3-c]pyridine, thieno[3,2-b]pyridine, thieno[2,3-b]pyridine, thieno[2,3-b]pyrazine, and the R1 group represents a methoxy or ethoxy, the R2 group represents a cyclohexylmethyl, a thiophene-2-ylmethyl, a thiophene-3-ylmethyl, a non-substituted benzyl, or a benzyl substituted by one or more F, Cl, OMe, CN.

3. The compounds of formula 1, according to claims 1 and 2 **characterized in that** they are selected from the following group:
3-Methoxythieno[2,3-b]pyridine-2-carboxylic acid (8-benzyl-8-azabicyclo[3.2.1]oct-3β-yl)amide
3-Methoxythieno[3,2-b]pyridine-2-carboxylic acid (8-benzyl-8-azabicyclo[3.2.1]oct-3β-yl)amide
3-Methoxyfuro[3,2-b]pyridine-2-carboxylic acid (8-benzyl-8-azabicyclo[3.2.1]oct-3β-yl)amide
3-Methoxythieno[3,2-c]pyridine-2-carboxylic acid (8-benzyl-8-azabicyclo[3.2.1]oct-3β-yl)amide
3-Methoxythieno[2,3-b]pyridine-2-carboxylic acid [8-(4-chlorobenzyl)-8-azabicyclo[3.2.1]oct-3β-yl]amide
3-Methoxythieno[2,3-b]pyridine-2-carboxylic acid [8-(4-methoxybenzyl)-8-azabicyclo[3.2.1]oct-3β-yl]amide
3-Methoxythieno[2,3-b]pyridine-2-carboxylic acid [8-(4-cyclohexylmethyl)-8-azabicyclo[3.2.1]oct-3β-yl]amide
3-Methoxythieno[2,3-b]pyridine-2-carboxylic acid [8-(4-fluorobenzyl)-8-azabicyclo[3.2.1]oct-3β-yl]amide
3-Methoxythieno[2,3-b]pyridine-2-carboxylic acid [8-thiophen-3-ylmethyl-8-azabicyclo[3.2.1]oct-3β-yl]amide
3-Methoxythieno[2,3-c]pyridine-2-carboxylic acid (8-benzyl-8-azabicyclo[3.2.1]oct-3β-yl)amide
3-Isopropoxythieno[2,3-b]pyridine-2-carboxylic acid (8-benzyl-8-azabicyclo[3.2.1]oct-3β-yl)amide
3-Ethoxythieno[2,3-b]pyridine-2-carboxylic acid (8-benzyl-8-azabicyclo[3.2.1]oct-3β-yl)amide
3-Methoxyfuro[3,2-b]pyridine-2-carboxylic acid [8-(4-fluorobenzyl)-8-azabicyclo[3.2.1]oct-3β-yl]amide
3-Methoxyfuro[3,2-b]pyridine-2-carboxylic acid [8-(4-chlororobenzyl)-8-azabicyclo[3.2.1]oct-3β-yl]amide
3-Methoxyfuro[3,2-b]pyridine-2-carboxylic acid (8-cyclohexylmethyl-8-azabicyclo[3.2.1]oct-3β-yl)amide
3-Methylsulfanylthieno[2,3-b]pyridine-2-carboxylic acid (8-benzyl-8-azabicyclo[3.2.1]oct-3β-yl)amide
3-Methoxythieno[2,3-b]pyrazine-2-carboxylic acid (8-benzyl-8-azabicyclo[3.2.1]oct-3β-yl)amide
3-Methoxythieno[2,3-b]pyrazine-2-carboxylic acid [8-(4-chlorobenzyl)-8-azabicyclo[3.2.1]oct-3β-yl]amide
3-Methylsulfanylthieno[2,3-b]pyrazine-2-carboxylic acid (8-benzyl-8-azabicyclo[3.2.1]oct-3β-yl)amide
3-Methylsulfanylthieno[2,3-b]pyrazine-2-carboxylic acid [8-(4-chlorobenzyl)-8-azabicyclo[3.2.1]oct-3β-yl]amide
3-Methoxythieno[2,3-b]pyrazine-2-carboxylic acid [8-(4-fluorobenzyl)-8-azabicyclo[3.2.1]oct-3β-yl]amide
3-Methylsulfanylthieno[2,3-b]pyrazine-2-carboxylic acid [8-(4-fluorobenzyl)-8-azabicyclo[3.2.1]oct-3β-yl]amide
3-Methoxythieno[2,3-b]pyrazine-2-carboxylic acid (8-thiophen-3-ylmethyl-8-azabicyclo[3.2.1]oct-3β-yl)amide
3-Methoxyfuro[2,3-b]pyrazine-2-carboxylic acid (8-benzyl-8-azabicyclo[3.2.1]oct-3β-yl)amide
3-Methoxyfuro[2,3-b]pyrazine-2-carboxylic acid [8-(4-chlorobenzyl)-8-azabicyclo[3.2.1]oct-3β-yl]amide
3-Methoxyfuro[2,3-b]pyrazine-2-carboxylic acid [8-(4-fluorobenzyl)-8-azabicyclo[3.2.1]oct-3β-yl]amide

4. Method to prepare the compounds of general formula 1, according to one of claims 1 to 3, **characterized in that** a heterocyclic acid of formula 2, 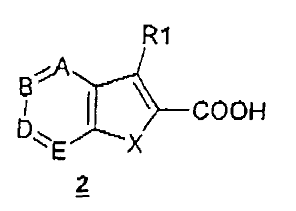 is allowed to react with a polycyclic amine of formula 3, 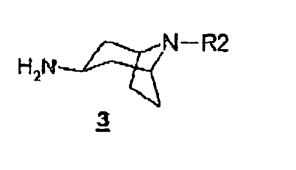 wherein the different radicals A, B, D, E, X, R1 and R2 have the significations given in claim 1, under coupling conditions with the action of alkyl chloroformiate in the presence of triethylamine, in methylene chloride, at a low temperature or by the action of the acid chloride of the corresponding heterocyclic acid.

5. The compounds of formula 4, as synthesis intermediates, wherein X is an O or S, R1 an OH, a C1-6 alcoxy, or a C1-6 thioalcoxy, and R represents a hydrogen, except in the case in which R1 is OH, a methyl or an ethyl.

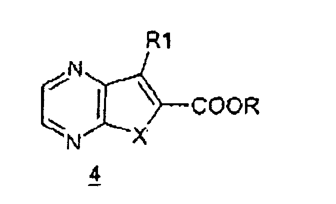

6. The compounds of formula 4, according to claim 5, **characterized in that** they are selected from:
3-Hydroxy-2-methoxycarbonylfuro[2,3-b]pyrazine
3-methylsulfanyl-2-methoxycarbonylfuro[2,3-b]pyrazine
3-methoxy-furo[2,3-b]pyrazine-2-carboxylic acid
3-ethoxy-furo[2,3-b]pyrazine-2-carboxylic acid
3-methylsulfanylfuro[2,3-b]pyrazine-2-carboxylic acid
3-Hydroxy-2-methoxycarbonylthieno[2,3-b]pyrazine
3-methylsulfanyl-2-methoxycarbonylthieno[2,3-b]pyrazine
3-methoxythieno[2,3-b]pyrazine-2-carboxylic acid
3-ethoxythieno[2,3-b]pyrazine-2-carboxylic acid
3-methylsulfanylthieno[2,3-b]pyrazine-2-carboxylic acid.

7. As a medicinal product, the compounds of general formula I according to one of claims 1 to 3.

8. Pharmaceutical formulation, **characterized in that** it comprises at least one compound of formula 1 according to one of claims 1 to 3, and a suitable excipient, particularly for oral or parenteral administration, particularly at a daily dose of 0.1 to 100 mg.

9. Use of a compound of formula 1 according to any of claims 1 to 3, for the preparation of a medicinal product intended to treat schizophrenia, the positive or negative symptoms thereof or disorders of the central nervous system sensitive to antidopaminergic treatment, such as obsessive-compulsive disorders, anxiety, depression, drug addiction, late dyskinesia, autism, gastrointestinal disorders.
